# EUROPEAN PATENT APPLICATION

(11) **EP 2 949 332 A2**
(43) Date of publication of application: **02.12.2015**
(21) Application number: 14743853.5
(22) Date of filing: 28.01.2014
(51) Int. Cl.: A61K 33/04, A61K 9/16, A61K 31/353

(54) **COMPOSITIONS FOR THE SYSTEMIC TREATMENT OF PATHOLOGICAL CONDITIONS RESULTING FROM OXIDATIVE STRESS AND/OR REDOX IMBALANCE**

(30) Priority: 28.01.2013 US 201313751429
(71) Applicant: Nuevas Alternativas Naturales Thermafat, S.A.P.I. de C.V., Monterrey, Nuevo León 64320 (MX)
(72) Inventor: GOJON ROMANILLOS, Gabriel, Nuevo León 66220 (MX)
(74) Representative: Carpmaels & Ransford LLP
(86) International application number: PCT/MX2014/000029
(87) International publication number: WO 2014/116097

(57) **Abstract**

Alterations of redox homeostasis in mammals underlie a host of symptoms, syndromes and diseases, including AIDS and cancer, which can be successfully treated by administration to a mammal of therapeutically-effective amounts of sulfide compounds and/or thiosulfate compounds and/or thionite compounds and/or thionate compounds and/or any chemical species capable of providing to a subject in need thereof a sulfide compound, thiosulfate compound, thionite compound, or thionate compound. The unique compositions of this invention contain one or more "active sulfur substances" in combination with each other or with other therapeutic agents. The invention also encompasses the varying modes of administration of the therapeutic substances. In particular, a novel method of combining active ingredient with wet cellulose is provided, which allows the wet cellulose to function as an enteric carrier.

## Description

### Cross-reference to related applications

This application claims the benefit of continuation-in-part of U.S. Application Ser. No. 13/751,429, filed on January 28, 2013, which is a continuation-in-part of U.S. Application Ser. No. 12/543,407, filed on August 18, 2009, which is a continuation-in-part of U.S. Application Ser. No. 12/405,165, filed March 16, 2009, which is a division of U.S. Application Ser. No. 10/463,765, filed June 18, 2003, which claim the benefit of U.S. Provisional Application Ser. No. 60/389,491, filed June 19, 2002. These applications are hereby incorporated by reference in their entirety.

### Background of the Invention

The present invention relates to novel compositions for and methods of treating symptoms, syndromes, pathological conditions and disease-associated problems mediated by oxidative stress. These conditions include cancer, AIDS, diabetes, cardiovascular diseases, Down syndrome, chronic inflammatory diseases, neurodegenerative diseases, cachexia secondary to HIV-1 infection, cachexia secondary to cancer and AIDS related complex (ARC), and hypercholesterolemia. Also included is the novel preparation of active ingredient with an enteric carrier.

Inorganic thiosulfates and sulfites have found wide use in pharmacology and/or in the formulation of final dosage forms as preservatives, antioxidants, or biocides. Thus, thiosulfates find application in the treatment of cyanide poisoning, allergic conditions and drug sensitization caused by gold, arsenic, mercury or bismuth preparations in humans, and in veterinary medicine as cyanide antidotes, as "general detoxifiers" and also in bloat and, externally, in treatment of ringworm or mange. Injection of aqueous solutions of sodium thiosulfate and L-cysteine or its sodium salt are claimed to be effective against "bacteria and viruses" in general. U.S. Pat. No. 4,148,885 issued to Renoux et al., discloses use of sodium thiosulfate and sodium metabisulfite as immunostimulants, but strictly within the context of "a process for stimulating the immunity of a living organism", although only mice are mentioned and only subcutaneous administration was employed.

Sulfites also display some pharmacological activity against the agents responsible for certain parasitic and infectious conditions. In addition German patent DE3419686 discloses sulfite or bisulfite solutions for treating arthritis or epilepsy. WO8402527 claims increased anti-tumor activity for adriamycin and daunomycin with the addition of sulfites, acid sulfites, pyrosulfites and/or dithionites. U.S. Pat. No. 5,045,316 issued to Kaplan, claims that a combination of an ionic vanadium compound, a thiosulfate or sulfite, and optionally selenium is useful for treating malignant tumors, atherosclerosis and mental syndromes in the elderly. However, it should be clear that in the prior art neither thiosulfates nor sulfites have been claimed to act as herein disclosed by themselves or in admixture with each other and/or with sulfide compounds, thionite compounds, or thionate compounds, when delivered to a mammal in need thereof.

It should also be appreciated that both thiosulfates and sulfites are rapidly decomposed when released in the stomach, so that oral administration of aqueous solutions, tablets, or capsules containing sulfites or thiosulfates cannot be used for their delivery to the gut of a mammal, unless an enteric coating, enteric carrier or other ad-hoc delivery system is employed. Exactly the same considerations apply to dithionites, which have been used (see above) in combination with adriamycin and daunomycin. On the other hand, sulfide compounds and thionate compounds have been, to the best of the present applicant's knowledge, neither claimed to act as herein disclosed nor hypothesized to be capable of such action when delivered to a mammal.

Without intending to be bound by any particular hypothesis or theory, current thinking on the etiology of cancer, AIDS, cardiovascular diseases, diabetes, Down syndrome, chronic inflammatory diseases and neurodegenerative disorders will be reviewed, in an attempt to understand the basis for the surprising success of the treatment method disclosed herein. Since the diverse sulfur containing substances found by the present applicant to be pharmacologically active all possess reducing properties, special attention will be given to the possibility that a link exists between the two sets of properties and to research that bears on oxidation-reduction processes in cells, especially if it focuses on oxidative stress or its pathological manifestations.

In healthy human tissue a delicate balance between cell proliferation and cell death exists, which when disrupted can lead to a degenerative disease (diabetes and its vascular complications, anemia, arthritis, Parkinson's disease, Alzheimer's disease, Amyotrophic Lateral Sclerosis (ALS), Huntington's disease, muscular dystrophy, myotonic dystrophy, chronic fatigue syndrome, Friedreich's ataxia, ocular lens opacification, nephrosis, liver necrosis, dermatitis, pulmonary immune deficits, hepatic encephalopathy, macular degeneration, age-associated memory impairment, Creutzfeldt-Jacob's disease, stroke, epilepsy, peripheral neuropathy, optic neuropathy, anatomic neuropathy, neurogenic bowel disease, sensorineural deafness, neurogenic bladder dysfunction, migraine, renal tubular acidosis, dilating cardiomyopathy, hepatic failure, lactic acidemia, arsenic poisoning, silicosis, acetaminophen poisoning, asbestosis, asthma, rheumatic polyarthritis, adult respiratory distress syndrome) in case of premature cell loss. Similarly, disruption of this balance can lead to a hyperproliferative disease (cancer, AIDS, herpes simplex virus-1 infection, cytomegalovirus-induced vascular pathology, arteriosclerosis, ARC, hepatitis, trypanosomiasis, vascular restenosis, psoriasis, glomerular nephritis, transplant rejection, etc.) in case of cell over-accumulation. It must be pointed out that mitochondrial function is the key to this balance, since mitochondria regulate apoptosis-the physiological mechanism for the elimination of cells in a controlled and timely manner.

The defense mechanism of a mammal (humoral/cellular immunity mediated by non-phagocytic lymphocytes, phagocytic polymorphonuclear leucocytes, and voraciously phagocytic monocytes/macrophages) eliminates foreign bodies such as microorganisms (bacteria, rickettsias, viruses, fungi, protozoa or metazoa) and abnormal cells, including neoformed cells capable of becoming a cancerous tumor such as a carcinoma, sarcoma, myoma or lymphoid tumor through hyperproliferation.

Cancerous tumors and hematological malignancies are usually life-threatening. In humans they include, among others, prostate, colon, breast, lung, kidney, liver, lymphoma of the central nervous system (CNS), leukemia, pancreatic, gastric, esophageal, ovarian, uterine, testicular and skin tumors. Most human and animal cancer involves cells of epithelial origin, whose malignant transformation results in carcinomas, i.e., tumors of epithelial cell origin.

The balance between cell proliferation and cell death in a healthy mammal depends critically on both an intact immune system, and a finely tuned systemic balance between antioxidants and oxidants, which will be referred to hereinafter as "redox homeostasis". Moreover, redox homeostasis is also essential for the components of the immune system to function adequately.

Stepwise reduction of molecular oxygen (dioxygen) to water inside mammalian cells is the source of the ATP needed by the cell to power its multiple activities. However, the partially reduced intermediates formed during this process (superoxide radical anion, hydrogen peroxide, hydroperoxy radical and hydroxy radical) are highly reactive and their leakage can be the cause of inflammation, oxygen toxicity, oxidative stress, and/or oxidative damage to biomolecules and complex cell structures such as membranes and mitochondria; these partially reduced species are known collectively as "reactive oxygen intermediates" (ROI's). Furthermore, some cells belonging to the immune system generate hypochlorous acid or ROI's ("respiratory burst") in order to use them as weapons against foreign bodies. Detoxication of xenobiotics (including drugs) is another common source of ROI's, as well as the enzymatic synthesis of prostaglandins, thromboxanes, and leukotrienes from polyunsaturated fatty acids in epithelial cells.

During the last decade, it has become evident that ROI's perform an extremely important direct role in signal transduction. Most sources of the ROI's involved in signal transduction seem to initially generate superoxide, whose disproportionation then yields hydrogen peroxide. As noted by Powis et al. ("Redox signaling and the control of cell growth and death", in Helmut Sies (ed.) "Antioxidants in disease mechanisms and therapy," Academic Press, 1997), intracellular redox signaling is the result of controlled changes in the intracellular redox state. This signaling can regulate the cell cycle, including the control of DNA synthesis, enzyme activation, and gene expression. The redox signaling operates by changing the conformation of key proteins by changing the oxidation state of cysteine residues in these proteins. These conformational changes affect the biological function of the protein. These conformationally sensitive proteins directly affect cell growth and differentiation, as well as cellular apoptosis.

A variety of experimental results, reported between 1994 and 2000, illustrate the importance of redox status/ROI's in cellular signaling systems and mammalian health. Metallothionein-III (MT-III) is a brain-specific metallothionein, which is markedly reduced in the brain of patients with Alzheimer's disease (AD) and other degenerative diseases. Oxidative stress seems to be one of the principal factors that modulate MT-III mRNA (Messenger Ribonucleic Acid) expression. Pulmonary surfactant, a mixture of phospholipids and surfactant proteins (SP-A and SP-B) reduces surface tension at the air-liquid interface and protects the large epithelial surface of the lung from infectious organisms. Cellular oxidants reduce surfactant protein expression. Also, antioxidants reduce cyclooxygenase-2 expression, prostaglandin production and proliferation in colorectal cancer.

Overexpression of mdr-1 type transporters in tumor cells contributes to multidrug-resistance. The induction of mdr-1 mRNA and of functionally active mdr1-type P-glycoprotein by elevation in intracellular levels of reactive oxygen species and the repression of intrinsic mdr-1 mRNA and P-glycoprotein overexpression by antioxidants support the conclusion that the expression of the mdr-1 b P-glycoprotein is regulated in a redox-sensitive manner.

Oxidative stress affects the expression of various regulatory genes in rabbit lens epithelial cells, which likely affects cell proliferation, differentiation, and viability and thus affects normal cell function.

In cultured keratinocytes, butylated hydroxytoluene hydroperoxide (BHTOOH) stimulates a time-dependent increase in ornithine decarboxylase (ODC) enzyme activity paralleled by induction of ODC mRNA (mRNA that directs ODC synthesis), suggesting transcription regulation of ODC by BHTOOH. Depletion of intracellular glutathione caused a 5-fold potentiation of keratinocyte sensitivity to BHTOOH and consequently, of tumor promotion.

ROIs can also act indirectly as signal transducers by modifying the bioavailability of nitric oxide (NO). Thus, inflammatory cytokines such as tumor necrosis factor-alpha (TNF-α) and interleukins (ILs) induce NO (nitric oxide) overproduction. NO is a messenger endogenously synthesized by a variety of mammalian cells including neurons, smooth muscle cells, macrophages, neutrophils, and platelets. In fact there is cross-talk between ROIs and NO, since the effects of the latter are influential on signaling pathways regulated by thiolic redox status.

However, if superoxide and NO interact a powerful non-radical oxidant, peroxynitrite (PN), is readily formed. PN is capable of oxidizing a number of biomolecules and complex cell structures including enzymes such as catalase and glutamine synthetase, proteins containing tyrosine residues, DNA, brain mitochondria and membrane lipids such as synaptosomal membranes.

NO itself has been implicated in a variety of neurodegenerative disorders and is a mediator in excitotoxic and post-hypoxic damage to neurons. DNA strand breakage is induced synergistically by NO and a catecholamine.

Most living organisms have evolved well-integrated antioxidant defense mechanisms, which include both antioxidant enzymes such as catalase, superoxide dismutases, glutathione peroxidases, quinone reductase, diaphorase and ceruloplasmin and low molecular weight antioxidants (LMWAOs) such as pyruvic acid, glutathione (GSH), dihydrolipoic acid (DHLA), beta-carotene, vitamin C, vitamin E and thioredoxin (TRX, a ubiquitous, relatively small, dithiolic, hydrogen-carrier protein).

Whereas antioxidant vitamins and beta-carotene must be supplied through food intake (e.g., in fruits and vegetables), both the thiolic tripeptide glutathione and DHLA are endogenous antioxidants, as well as pyruvic acid.

Pyruvic acid, being a normal tissue metabolite, is likely to be non-toxic and its high effectiveness as a "peroxide scavenger" is well documented; furthermore, after scavenging hydrogen peroxide or organic hydroperoxides it is converted into acetic acid, which means that it is intrinsically incapable of acting as a prooxidant. In spite of these attributes, pyruvic acid's role as an endogenous antioxidant has been widely underestimated: it is probably an important but underrated contributor to the "redox buffering" capacity of blood serum.

Glutathione (L-gamma-glutamyl-L-cysteinylglycine) is a ubiquitous intracellular thiol present in almost all mammalian tissues; the liver has very high intracellular levels of GSH.

Besides maintaining cellular integrity by enforcing a reducing environment, GSH has multiple functions including detoxification of xenobiotics; synthesis of proteins, nucleic acids, leukotrienes, prostaglandins and thromboxanes through its action as a coenzyme; and preventing other antioxidants from becoming pro-oxidants.

GSH enforces a reducing intracellular environment by acting as an excellent scavenger of both oxygen-centered and nitrogen-centered free radicals (reactive nitrogen intermediates, RNIs) and by readily converting non-radical oxidants (PN, peroxides, hydroperoxides) into harmless compounds. After acting as a coenzyme or scavenging ROI's or PN, GSH is oxidized to GSSG (glutathione disulfide), from which GSH is regenerated enzymatically. The redox system of GSH consists of primary and secondary antioxidants, including glutathione peroxidases, glutathione reductase, glutathione-S-transferase, and glucose-6-phosphate dehydrogenase.

Redox reactions in which GSH plays a role include protein folding, conversion of ribonucleotides to deoxyribonucleotides, and maintenance of reduced pools of vitamins C and E; GSH can also undergo reversible thiol-disulfide exchange with proteins containing oxidized cysteine (i.e., cystine) residues.

Whereas in tissues and red blood cells (CA 131:156247v) GSH is the foremost "redox buffer," in blood plasma this function has been assigned to albumin, although the applicants believe that pyruvic acid is also a key antioxidant in both environments.

As stated above, DHLA and thioredoxin perform roles that complement those of GSH; their oxidized forms can also be reduced easily by enzyme action. Vitamins C and E, which can readily and reversibly act as hydrogen donors as well, also contribute to maintain the intracellular oxidant-reductant balance (redox homeostasis).

By operating in a concerted and often synergistic manner, the redox mediators GSH, DHLA, TRX, Vitamin C, Vitamin E, and the antioxidant enzymes help maintain a reducing intracellular environment. This reducing environment performs a variety of important cellular functions. First, it helps keep bioactive quinones in the reduced state. For example, cardiotonic ubiquinones and vitamin K are maintained in their reduced state (ubiquinol/hydroquinone), so as to minimize the probability of arylating DNA and of generating ROI's in anaerobic or aerobic conditions. Also, it keeps catecholamines (adrenaline, dopamine, etc.) in the reduced (hydroquinone) condition, preventing their irreversible oxidation to quinoneimines of the adrenochrome type. The reducing intracellular environment also prevents vasoactive serotonin from being oxidized to a reactive quinoneimine.

The reducing intracellular environment prevents inactivation of heart dihydrolipoamide dehydrogenase and of other oxidant-sensitive enzymes such as glutamine synthetase. The reducing environment attenuates hypersensitivity responses induced by oxidative activation of phenolic haptens, and preserves the functional integrity of the blood-brain barrier, of the intestinal epithelium, and of the heart endothelium. It also helps preserve cytoskeletal integrity. The reducing environment protects synaptosomal membranes from oxidation, and prevents the death of hippocampal neurons. It is also important to phagocytes, as it supports their random migration, chemotaxis, ingestion and superoxide production.

Of particular significance is the role of a reducing environment in preserving the functional integrity of mitochondria. GSH and glutathione peroxidase (GPx) play a critical role here, since mitochondria lack catalase, an enzyme which degrades hydrogen peroxide. "Mitochondrial diseases" are disorders to which deficits in mitochondrial respiratory chain activity contribute. This category includes deficiencies in the activity of components of the mitochondrial respiratory chain. Typically, these deficiencies are caused by exposure of the cells to nitric oxide and hypoxia or ischemia or oxidative stress on the tissue. These deficiencies in antioxidants or antioxidant enzymes can result in or exacerbate mitochondrial degeneration. It must be pointed out that redox homeostasis also requires a delicate antioxidant enzyme balance in cells: too much Superoxide Dismutase (SOD) relative to GPx or catalase results in the accumulation of hydrogen peroxide, which in turn, through the Fenton reaction, leads to the production of hydroxyl radical and concomitant cell damage. However, too little SOD enzyme is also not favorable because superoxide radicals in themselves are toxic to cells. Therefore, fine-tuning of the antioxidant enzymes (together with the nonenzymatic antioxidants) becomes imperative if the cell is to function successfully in an oxygen-rich environment.

Down syndrome is believed to be the consequence of a congenital perturbation in the balance of antioxidant enzymes, with damage to important biomolecules brought about by a highly pro-oxidant intracellular environment.

In the face of stresses such as injury or infection, organisms rapidly marshal a host of responses: immune cells are recruited and various genes are rapidly activated. The key coordinating factor in this activation is the nuclear transcription factor NF-κB, which also plays a crucial role in modulating gene expression during growth and development.

Among the genes modulated by NF-κB are those encoding cytokines (TNF-α, ILs, etc.) and growth factors, immunoreceptors, adhesion molecules, acute-phase proteins, other transcription factors and regulators, NO-synthase, and viral genes. Most target genes for NF-κB are intrinsically linked to a coordinated inflammatory response.

NF-κB has far-reaching significance for a variety of pathological conditions in which chronic inflammation, growth, or viral activation occur, such as tumorigenesis, HIV infection (AIDS), atherosclerosis, diabetes, rheumatoid arthritis, chronic bronchitis, cystic fibrosis, idiopathic pulmonary fibrosis, ARDS, septic shock, cirrhosis, ulcerative colitis, reperfusion injury, inflammatory bowel disease, pulmonary emphysema, neurodegenerative disorders, (Alzheimer's Disease, Parkinson's Disease, etc.), osteoporosis, asthma, renal disease, rheumatoid synovitis, and the animal model of multiple sclerosis, experimental allergic encephalomyelitis.

An important NF-κB regulated gene is that encoding the cytokine TNF-α which plays a central role in several inflammatory conditions. Since TNF-α is itself an activator of NF-κB, the potential for a positive inflammatory feedback cycle with disastrous consequences can be envisioned.

As stated above, the activation of NF-κB has been implicated in a wide range of diseases in which there is an inflammatory and/or hyperproliferative component including AIDS, where the expression of HIV is NF-κB dependent. It is now clear that ROI/RNI are mediators of NF-κB activation, and also that this process can be blocked by antioxidant agents.

Antioxidant agents can also inhibit the production of TNF-α. Excessive or unregulated TNF-α production mediates or exacerbates a number of diseases including rheumatoid arthritis, rheumatoid spondylitis, osteoarthritis, gouty arthritis, sepsis, septic shock, endotoxic shock, gram-negative sepsis, toxic shock syndrome, ARDS, cerebral malaria, chronic pulmonary inflammatory disease, silicosis, asbestosis, pulmonary sarcoidosis, bone resorption diseases, reperfusion injury, graft vs. host reaction, allograft rejections, cachexia secondary to infection or malignancy, cachexia secondary to acquired immune deficiency syndrome (AIDS), AIDS related complex, keloid formation, scar tissue formation, Crohn's disease, ulcerative colitis, pyrosis and fever and myalgias due to infection.

Although most experts would admit the possibility that the time course and even the final outcome, of a disease can be critically modulated by strengthening the antioxidant side of the balance between prooxidants and antioxidants, it is unfortunately true that single antioxidants as pharmacologically active agents have not been found to exhibit extremely powerful therapeutic effects. For example, the jury is still out regarding the effectiveness of vitamin C as a therapeutic agent. Nevertheless, vitamin C may have a role in impeding the progress of diabetes, cataract, heart disease, cancer, aging, and a variety of other disease states. Several methods for modulating cellular GSH levels in human diseases associated with GSH deficiency and oxidative stress are still being evaluated. DHLA, regarded in some quarters as a unique "ideal" antioxidant remains an intriguing possibility for the treatment of conditions (notably AIDS, atherosclerosis and diabetes) related to oxidative stress.

Treatment of severe vitamin E deficiency with appropriate supplements of the vitamin can at least halt the progression of the characteristic neurological features, but in the majority of clinical neurological conditions the therapeutic benefits of antioxidant supplementation still requires to be proved. On the other hand, vitamin E has been reported to regress oral leukoplakia (a precancerous lesion). Supplemental beta-carotene reduces the frequency of "oral micronuclei" (an indicator of genotoxic damage to the oral epithelium) significantly; it is also effective against oral leukoplakia. Preliminary results of studies on pre-cachectic and cachectic HIV-infected patients indicate that the decrease of plasma cystine, glutamine, and arginine levels can be corrected by N-Acetyl-L-Cysteine (NAC). Anecdotal data also suggest that this strategy may slow or even prevent the progression of the disease.

A preliminary report indicates that ARDS patients receiving NAC (by mouth), alpha-tocopherol (by mouth), selenium (iv) and ascorbic acid (iv) within 24 hrs of diagnosis for 3 days experienced a significant reduction in mortality (n=25; 20% mortality) compared to a control group (n=20; 65% mortality); however, these results are in need of validation.

Finally, a randomized trial (n=65) with biopsy-confirmed transitional cell bladder carcinoma patients yielded promising results: the 5-year estimate of tumor recurrence was 91 % in the "RDA arm" (patients receiving multivitamins at RDA (Recommended Daily Allowance) levels) vs. 41% in the "megadose arm" (patients receiving multivitamins at RDA levels plus 40,000 IU retinol plus 100 mg pyridoxine plus 2000 mg ascorbic acid plus 4000 IU alpha tocopherol plus 90 mg zinc).

This rather limited success might seem at first surprising in view of the decreased levels of selected major antioxidants consistently found in a number of disease states (GSH in AIDS, hepatitis C, type II diabetes, ulcerative colitis, ARDS, idiopathic pulmonary fibrosis and neurodegenerative syndromes; vitamin E in atherosclerosis, ARDS, Down syndrome and Alzheimer's disease; ascorbic acid in ARDS; beta-carotene in cystic fibrosis; vitamin A in Down syndrome and Alzheimer's disease, etc.). The limited success of this "magic single antioxidant approach" can be rationalized, however, by recalling that mammals possess highly evolved and well-integrated antioxidant mechanisms which require the concerted and synergistic action of both antioxidant enzymes and low molecular weight antioxidants, with different antioxidants operating extracellularly and/or in specific cell compartments (aqueous vs. lipidic microenvironments) and having limited functional overlap. Some antioxidants destroy peroxidic species and/or PN, others break free radical chains, still others quench singlet oxygen.

There are other foreseeable obstacles in the way of the "single antioxidant" approach to therapy. Several antioxidants have been shown to be capable of acting as pro-oxidants or as NF-κB activators "in vitro" and/or "in vivo" under rather specific conditions, including ascorbic acid, beta carotene, glutathione, flavonoids, NAC, and L-cysteine. Limited evidence suggests that administration of a single antioxidant might have adverse effect(s) on plasma levels of other antioxidants.

After this appraisal of the current biochemical research on the etiology of cancer, AIDS, cardiovascular diseases, diabetes, Down syndrome, neurodegenerative disorders and chronic inflammatory diseases, the following hypotheses might help explain the remarkable success of the therapy system herein described:
(i) The sulfur containing substances comprised by the therapy system herein disclosed act as inducers of antioxidant enzymes, thereby enhancing the immune system and/or reactivating mitochondria and/or increasing GSH levels, i.e. their effects are similar to those of 1,2-dithiole-3-thiones. (ii) The sulfur containing substances herein disclosed act as powerful antioxidant enzyme activators. Specifically, they interact chemically, as reductants, with inactivated enzymes containing disulfide bonds which are thereby cleaved and converted into thiol groups with concomitant restoration of enzyme function. In this case their effect would be akin to that of hydrogen sulfide on inactivated (oxidized) papain. (iii) The "active sulfur substances" herein described act (directly or indirectly) as powerful inhibitors of transcription factor NFkB and TNF alpha. (iv) The sulfur containing substances on which the present invention is based act indirectly through the delivery of "reducing equivalents" to cells subject to oxidative stress, their effect being restoration of redox homeostasis and immune function. The mediator might well be pyruvic acid (see above) since it is known that pyruvic acid can act systemically when delivered to the gut; i.e. it can be readily transported from the gut to other tissues. Further, pyruvate has been shown to enhance the endogenous GSH system. Also, there is a linear relationship between GSSG-to-GSH and lactate-to-pyruvate ratios in human blood before, during and after exercise.

In a study on the nutritional requirements of human gut sulfate-reducing bacteria, it was found that short-chain fatty acids such as butyric acid, lactic acid, and other organic acids, alcohols, and amino acids (but not sugars or aromatic compounds) stimulated sulfate reduction. Experiments with two strains of Desulfovibrio desulfuricans isolated from human feces demonstrated that both were able to reduce sulfite, thiosulfate or nitrate in the absence of sulfate.

Therefore, while the present invention is not to be restricted by any hypothesis, it is possible that pyruvate, synthesized in the gut by bacterial microflora from lactate and sulfite or thiosulfate (or some other sulfur species capable of undergoing reduction), is then transported to the mammal's tissues, wherein it acts, mainly at the mitochondrial level, as a peroxide scavenger and a source of both NADH and energy (via acetyl coenzyme A). NADH (reduced nicotinamide adenine dinucleotide) can then enzymatically reduce lipoic acid (LA) to DHLA, which can in turn reduce GSSG to GSH.

Considering the seriousness of the AIDS pandemic, the global burden of cancer (with close to 10 million newly diagnosed cases each year), and the devastating effects of such diseases as diabetes, chronic inflammatory diseases, neurodegenerative pathologies, and Down syndrome it is clear that a pressing need exists for effective treatments of pathological states related to oxidative stress and/or exacerbated or mediated by NF-κB/TNF-α, such as the ones referred to above. This becomes even more clear when we consider the fact that cardiovascular diseases (and atherosclerosis, which is believed to be their underlying primary cause) are the main cause of death in most developed countries.

### Summary of the Invention

The invention features a pharmaceutical composition in unit dosage form for oral administration, the composition including an active sulfur substance, wherein the active sulfur substance is capable of providing to a subject in need thereof a therapeutically effective or prophylactically effective amount of a sulfide compound, thionite compound, thionate compound, thiosulfate compound, or a hydrate, salt, or mixture thereof; and optionally an enteric carrier.

The invention also features a pharmaceutical composition for parenteral administration, the composition including an active sulfur substance, wherein the active sulfur substance is a chemical species capable of providing to a subject in need thereof a therapeutically effective or prophylactically effective amount of a sulfide compound, thionite compound, thionate compound, thiosulfate compound, or hydrates, salts, and mixtures thereof, and one or more pharmaceutically-acceptable carriers, diluents and adjuvants.

In certain aspects, the pharmaceutical composition includes an active sulfur substance selected from the group consisting of a sulfide compound, thionite compound, thionate compound, thiosulfate compound, or a hydrate, salt, or mixture thereof; and an enteric carrier in capsule form capable of delivering the active sulfur substance to the lower gastrointestinal tract of a subject upon ingestion without substantial release of the active sulfur substance in the stomach of the subject.

In other aspects, the composition includes a third agent and the third agent is selected from the group consisting of vitamins, micronutrients, coenzyme Q10, glucosamine, chondroitin sulfate, triiodothyronine, vinpocetine, pramiracetam, piracetam, hydergine, choline, niar, gallic acid, diallyl sulfide, anti-cancer agents, immunostimulants, antibiotics, hormone antagonists, antiviral agents, antihypertension agents, insulin, and anti-inflammatory agents.

In particular embodiments, the active sulfur substances are present in an amount from about 50 mg to about 1500 mg. In other embodiments, the unit dosage form is a hard gelatin capsule. In other embodiments, the unit dosage form is an acid-resistant capsule selected from the group consisting of: acid-armor capsules™ and DRcaps acid-resistant capsules™.

In one particular embodiment, the composition includes about 214 parts by weight sodium hydrogen sulfide (NaSH), about 640 parts by weight distilled water, and about 2000 parts by weight food grade microcrystalline cellulose.

In a second particular embodiment, the composition includes about 372 parts by weight sodium thiosulfate (Na₂S₂O₃), about 640 parts by weight distilled water, and about 2000 parts by weight food grade microcrystalline cellulose.

In all embodiments of the invention, the enteric carrier is selected from the group consisting of: wet microcrystalline cellulose, wet powdered cellulose, hypromellose, cellulose acetate-phthalate, gellan gum, methyl acrylate-methacrylic acid copolymers, cellulose acetate succinate, hydroxyl propyl methyl cellulose phthalate, hydroxyl propyl methyl cellulose acetate succinate, polyvinyl acetate phthalate, methyl methacrylate-methacrylic acid copolymers, sodium alginate, and stearic acid.

The invention further features a method of treating or preventing a disease associated with oxidative stress, the method including administering to a subject in need thereof a pharmaceutical composition in unit dosage form for oral administration, the composition including an active sulfur substance, wherein the active sulfur substance is capable of providing to a subject in need thereof a therapeutically effective or prophylactically effective amount of a sulfide compound, thionite compound, thionate compound, thiosulfate compound, or a hydrate, salt, or mixture thereof; and optionally an enteric carrier.

In one aspect, the invention features a method of treating a disease associated with oxidative stress, the method including providing to a subject in need thereof a therapeutically effective amount of an active sulfur substance selected from the group consisting of sulfide compounds, thionite compounds, thionate compounds, thiosulfate compounds, hydrates, salts, and mixtures thereof.

In another aspect, the invention features a method of preventing a disease associated with oxidative stress, the method including providing to a subject in need thereof a prophylactically effective amount of an active sulfur substance selected from the group consisting of sulfide compound, thionite compound, thionate compound, thiosulfate compound, hydrates, salts, and mixtures thereof.

In another aspect, the invention features a method of overcoming the nutritional deficits found in subjects suffering from sulfur deficiencies, the method including providing to a subject in need thereof an effective amount of an active sulfur substance selected from the group consisting of sulfide compound, thionite compound, thionate compound, thiosulfate compound, hydrates, salts, and mixtures thereof.

In yet another aspect, the invention features a method of administering an active sulfur substance to a subject in need thereof by formulating the active sulfur substance with an enteric carrier in capsule form, wherein the active sulfur substance is selected from the group consisting of sulfide compounds, thionite compounds, thionate compounds, thiosulfate compounds, hydrates, salts, and mixtures thereof and wherein the enteric carrier is capable of delivering the active sulfur substance to the lower gastrointestinal tract of the subject upon ingestion without substantial release of the active sulfur substance in the stomach of the subject.

In a final aspect, the invention features a method of delivering an active sulfur substance to a subject in need thereof by any means that leads to contact of the active sulfur substance with its site of action in the subject's body.

In preferred embodiments, the sulfide compound is hydrogen sulfide. In all embodiments of the invention, the subject is a mammal.

### Definitions

"Active sulfur substances", as defined in the current invention, encompass: 1) sulfide compounds, 2) thiosulfate compounds, 3) thionate compounds, 4) thionite compounds, and 5) chemical species capable of providing to a subject in need thereof a therapeutically effective or prophylactically effective amount of a sulfide compound, thiosulfate compound, thionite compound, or thionate compound. Non-limiting examples of such chemical species include elemental sulfur, which is known to be biotransformed into a sulfide compound (i.e., hydrogen sulfide) and/or conjugates which are known to be chemically and/or enzymatically transformed to sulfide compounds, thiosulfate compounds, thionite compounds, and/or thionate compounds.

As used herein "sulfide compounds" are compounds formally containing the divalent Sn moiety (S=sulfur; n=1, 2, 3 ...) chemically bonded to hydrogen and/or a metal (or metals) and/or a polyatomic cation (or cations) such as hydrogen sulfide, hydrogen disulfide, hydrogen tetrasulfide, sodium hydrosulfide, sodium hydrosulfide dihydrate, sodium sulfide, sodium sulfide nonahydrate, potassium sulfide, calcium sulfide, iron(II) sulfide, silicon (IV) sulfide, zinc sulfide, bismuth (III) sulfide, sodium disulfide, magnesium disulfide, iron (II) disulfide, sodium tetrasulfide, barium tetrasulfide, potassium pentasulfide, cesium hexasulfide, potassium iron (III) sulfide, ammonium sulfide, ammonium disulfide, ammonium tetrasulfide, and the like.

As used herein "thiosulfate compounds" are compounds formally containing the divalent thiosulfate moiety (S₂O₃) chemically bonded to hydrogen and/or a metal (or metals) and/or a polyatomic cation (or cations), such as sodium thiosulfate (Na₂S₂O₃), potassium thiosulfate (K₂S₂O₃), sodium thiosulfate pentahydrate (Na₂S₂O₃·5H₂O), magnesium thiosulfate (MgS₂O₃), silver thiosulfate (Ag₂S₂O₃), ammonium thiosulfate ((NH₄)₂S₂O₃), and the like.

As used herein "thionate compounds" are compounds formally containing the divalent SₙO₆ (wherein n>1) moiety chemically bonded to hydrogen and/or a metal (or metals) and/or a polyatomic cation (or cations), such as calcium dithionate (CaS₂O₆), barium dithionate dihydrate (BaS₂O₆·2H₂O), sodium trithionate, sodium tetrathionate and the like.

As used herein "thionite compounds" are compounds formally containing the divalent SₙO₂ₙ (wherein n=1 or 2) moiety chemically bonded to a hydrogen and/or a metal (or metals) and/or a polyatomic cation (or cations) such as zinc sulfoxylate, zinc dithionite, sodium dithionite, sodium dithionite dihydrate, and the like.

By "dietary supplement" is meant an agent, substance, and/or mixture of substances that is a food supplement or nutritional supplement intended to supplement the diet and provide nutrients, such as vitamins, minerals, fiber, fatty acids, or amino acids that may be missing or may not be consumed in sufficient quantities in a person's diet.

By "promote or maintain general health" is meant to aid in accomplishing a state of human health that is characterized by homeostatic balance with the stable condition of properties such as temperature, pH, electrolytes, and/or metabolites.

By "pharmaceutical composition" is meant a system comprising a substance described herein, optionally formulated with an acceptable excipient, and manufactured or sold with the approval of a governmental regulatory agency as part of a therapeutic regimen for the treatment of disease in a mammal or to promote and maintain general health. Pharmaceutical compositions can be formulated, for example, for oral administration in unit dosage form (e.g., a tablet, capsule, e.g., an acid-armor capsule™ sold by Arthur Andrew Medical, Inc, and/or a DRcaps acid -resistant capsule™ available from Capsulgel Inc., caplet, gel cap, or syrup); for topical administration (e.g., as a cream, gel, lotion, or ointment); for intravenous administration (e.g., as a sterile solution or colloidal dispersion free of particulate emboli and in a solvent system suitable for intravenous use); or in any other formulation described herein

As used herein, a "therapeutically-effective amount" refers to that amount that must be administered per day to a patient (a human or non-human mammal) in order to achieve an anti-tumor effect; to modulate an immune response; to modulate gene expression; to ameliorate Down syndrome; to treat hypercholesterolemia; to treat inflammatory gastrointestinal disorders; to treat hyperproliferative diseases other than cancer; to treat metabolic syndrome; to treat leukemia; to resolve inflammation; to treat diabetes or to treat cardiovascular disease. Methods of determining therapeutically effective amounts are well known.

As used herein, a "prophylactically-effective amount" refers to an amount that can be administered to a patient (e.g., a human or a non-human mammal) to delay, or preferably prevent the onset of the clinical disease. A prophylactically-effective amount can be administered to a patient with a clinically determined predisposition or increased susceptibility to development of cardiovascular diseases, cancer, diabetes, neurodegenerative diseases, AIDS, and other pathological conditions associated with oxidative stress, chronic inflammation, an imbalance in redox homeostasis, and/or immune dysfunction.

By "safe and effective amount" is meant the quantity of a composition which is sufficient to elicit a desired therapeutic and/or prophylactic response without undue adverse side effects (such as toxicity, irritation or allergic response) commensurate with a reasonable benefit/risk ratio when used in the manner of this invention.

As used herein, a "pharmaceutically acceptable component" is one that is suitable for use with humans and/or non-human mammals without undue adverse side effects (such as toxicity, irritation and allergic response) commensurate with a reasonable benefit/risk ratio. A pharmaceutically acceptable component is any ingredient other than the substance described herein (for example, a vehicle capable of suspending or dissolving the active substance and/or substances, e.g., petroleum jelly and polyethylene glycol) and having the properties of being nontoxic and non-inflammatory in a patient. Excipients may include, for example: antiadherents, antioxidants, binders, coatings, compression aids, disintegrants, dyes (colors), emollients, emulsifiers, fillers (diluents), film formers or coatings, flavors, fragrances, glidants (flow enhancers), lubricants, preservatives, printing inks, sorbents, suspending or dispersing agents, colloid stabilizers, sweeteners, and water. Excipients include, but are not limited to: butylated hydroxytoluene (BHT), calcium carbonate, calcium phosphate (dibasic), calcium stearate, croscarmellose, crosslinked polyvinyl pyrrolidone, citric acid, crospovidone, ethylcellulose, gelatin, hydroxypropyl cellulose, hydroxypropyl methylcellulose, lactose, magnesium stearate, maltitol, mannitol, methylcellulose, methyl paraben, microcrystalline cellulose, polyethylene glycol, polyvinyl pyrrolidone, povidone, pregelatinized starch, propyl paraben, shellac, silicon dioxide, sodium carboxymethyl cellulose, sodium starch glycolate, sorbitol, starch (corn), stearic acid, sucrose, talc, titanium dioxide, and xylitol. Excipients may also include diluents (e.g., saline and aqueous buffer solutions), aqueous carriers, and nonaqueous carriers, for example, water, ethanol, polyols (such as glycerol, propylene glycol, polyethylene glycol, and the like), and suitable mixtures thereof, vegetable oils, such as olive oil, and injectable organic esters, such as ethyl oleate.

By "enteric carrier" is meant an agent or compound added to the formulations described herein that allows the active ingredient(s) described herein (e.g., active sulfur substances) to pass through the stomach and be absorbed in the lower gastrointestinal tract. An enteric carrier is an agent or compound that protects against the effects of stomach juices, which can interact with, destroy, or degrade the active ingredient(s) described herein. Examples of enteric carriers include but are not limited to: wet microcrystalline cellulose, wet powdered cellulose, hypromellose, cellulose acetate-phthalate, gellan gum, methyl acrylate-methacrylic acid copolymers, cellulose acetate succinate, hydroxyl propyl methyl cellulose phthalate, hydroxyl propyl methyl cellulose acetate succinate, polyvinyl acetate phthalate, methyl methacrylate-methacrylic acid copolymers, sodium alginate, and stearic acid.

As used herein, "combination therapy" means that the patient (or non-human mammal) in need of treatment according to the present invention, is given medication not herein contemplated in addition to that herein disclosed. Combination therapy can be sequential therapy where the patient or non-human mammal is treated first with one or more drugs and then the other(s), or simultaneous therapy, when all drugs are co-administered.

By "treating" is meant subjecting a patient to a management regimen for the purpose of combating a disease or disorder and obtaining beneficial or desired results, such as clinical results. Beneficial or desired results can include, but are not limited to, resolution of inflammation, improvement in quality of life, alleviation or amelioration of one or more symptoms or conditions; diminishment of extent of disease, disorder, or condition; stabilization (i.e., not worsening) of a state of disease, disorder, or condition; prevention of spread of disease, disorder, or condition; delay or slowing the progress of the disease, disorder, or condition; amelioration or palliation of the disease, disorder, or condition; and remission (whether partial or total), whether detectable or undetectable

The term "mammals" is intended to mean both human and non-human mammals.

As used herein, "gut" means the region encompassed by the lower gastrointestinal tract, which includes the small intestine and its parts (e.g., the duodenum, jejunum, and ileum), the large intestine and its parts (e.g., the caecum, colon, and rectum), and/or the anus. The lower gastrointestinal tract excludes components of the upper gastrointestinal tract including the esophagus, stomach, and part of the duodenum.

By "delivering" is meant to provide and/or administer the active ingredient(s)described herein (a) as a solution or dispersion of the active ingredient(s) by enteroclysis, intravascularly, intravenously, intramuscularly, intrathecally, or subcutaenously; (b) oral administration of enterically coated tablets, granules, capsules, etc. which contain the active ingredient(s) and (optionally) one or more carriers and/or diluents and/or adjuvants. The composition may be administered in the form of tablets coated with an enteric coating; capsules having a shell, a filling comprising the active ingredient, and an enteric coating on the shell; or enterically coated granules comprising the active sulfur substance. The enterically coated granules may be included within a tablet, or as a filling within a capsule; (c) by oral administration of non enterically-coated capsules containing the active ingredient(s) and (optionally) one or more carriers and/or diluents and/or adjuvants intimately admixed with, and/or absorbed into, and/or adsorbed onto, an enteric carrier, such as wet microcrystalline cellulose; (d) by oral administration of delayed-release formulations containing the active ingredient(s) and (optionally) one or more carriers, diluents and adjuvants; (e) by rectal administration, as by using suppositories containing the active ingredient(s) and (optionally) one or more carriers, diluents and adjuvants; (f) transdermally; (g) transmucosally; or (h) by coadministration of the active ingredient (s) with any other pharmacologically active agents such as vitamins, micronutrients, coenzyme Q10, glucosamine, chondroitin sulfate, triiodothyronine, vinpocetine, pramiracetam, piracetam, hydergine, choline, niar, gallic acid, diallyl sulfide, anti-cancer agent (s), immunostimulant (s), antibiotic(s), hormone antagonist (s), antiviral agent(s), antihypertension agent(s), insulin and anti-inflammatory agent (s), optionally including one or more vehicles, carriers, diluents and adjuvants either orally (as in (b), (c), or (d) above) or by enteroclysis (as in (a) above) or rectally (as in (e) above).

"Parenteral administration" to a patient (or non-human mammal) includes but is not limited to: a) intravascular administration of solutions/dispersions containing at least one of the substances herein disclosed and, optionally, other active agents and/or one or more adjuvants; b) intramuscular administration of solutions/dispersions containing at least one of the substances herein disclosed and, optionally, other active agents and/or one or more adjuvants; c) subcutaneous administration of solutions/dispersions containing at least one of the substances herein disclosed and, optionally, other active agents and/or one or more adjuvants; d) intrathecal administration of solutions/dispersions containing at least one of the substances herein disclosed and, optionally, other active agents and/or one or more adjuvants; e) transdermal administration of appropriate formulations containing at least one of the substances herein disclosed and, optionally, other active agents and/or one or more adjuvants; f) transmucosal administration of appropriate formulations containing at least one of the substances herein disclosed and, optionally, other active agents and/or one or more adjuvants; g) sublingual administration of appropriate formulations containing at least one of the substancess herein disclosed and, optionally, other active agents and/or one or more adjuvants.

By "disease-associated problem" is meant a health problem derived from a specific disease, such as "cachexia secondary to cancer" or "muscle degeneration secondary to AIDS."

As used herein "disease mediated by oxidative stress" means health conditions related to a failure of cells to maintain redox homeostasis, which leads to oxidative damage by ROI's (Reactive Oxygen Intermediates) and RNI's (Reactive Nitrogen Intermediates). When the finely tuned balance between antioxidants and oxidants is tilted in favor of the latter, biomolecules, cell membranes and mitochondria are damaged, the immune system is compromised, cell-signaling and inflammation go awry and since mitochondria regulate apoptosis, the outcome is usually either a degenerative disease (diabetes and its vascular complications, anemia, arthritis, Parkinson's Disease, Alzheimer's Disease, amyotrophic lateral sclerosis, Huntington's Disease, muscular dystrophy, myotonic dystrophy, chronic fatigue syndrome, Friedreich's ataxia , ocular lens opacification, nephrosis, liver necrosis, pulmonary immune deficits, hepatic encephalopathy, macular degeneration, age-associated memory impairment, Creutzfeldt-Jacob's Disease, stroke, epilepsy, peripheral neuropathy, optic neuropathy, anatomic neuropathy, neurogenic bowel disease, sensorineural deafness, neurogenic bladder dysfunction, migraine, renal tubular acidosis, dilating cardiomyopathy, hepatic failure, lactic acidemia, arsenic poisoning, silicosis, acetaminophen poisoning, asbestosis, asthma, rheumatic polyarthritis, adult respiratory distress syndrome, etc.) or a hyperproliferative disease such as cancer, AIDS, herpes simplex virus-1 infection, cytomegalovirus-induced vascular pathology, arteriosclerosis, ARC, hepatitis , trypanosomiasis, vascular restenosis, psoriasis, glomerular nephritis, and/or transplant rejection. Diseases mediated by oxidative stress also include mitochondrial diseases defined herein as disorders which are characterized by deficits in mitochondrial respiratory chain activity . This category includes: a) congenital genetic deficiencies in activity of one or more components of the mitochondrial respiratory chain, and b) acquired deficiencies in the activity of one or more components of the mitochondrial respiratory chain, wherein such deficiencies are caused by, inter alia, oxidative damage during aging, and/or exposure of affected cells to NO.

As used herein, the term "about" means ±20% of the recited value.

Finally, a number of abbreviations are used in this application. The abbreviation ASK, as used herein, relates to the protein apoptosis signal-regulating kinase. The abbreviation IU refers to International Unit. The abbreviation LTR relates to the phrase long terminal repeat, while mdr relates to multiple drug resistance.

### Detailed Description

The present applicants serendipitously and unexpectedly discovered a therapy system useful for treating cancer, AIDS, cardiovascular diseases, Down syndrome, chronic inflammatory diseases, diabetes, neurodegenerative diseases and other disease states mediated or exacerbated by oxidative stress. This system comprises the delivery to the gut of a mammal of therapeutically effective amounts of one or more of the following active agents: sulfide compounds, thiosulfate compounds, thionite compounds, thionate compounds, and any chemical species capable of providing to a subject in need thereof a sulfide compound, thiosulfate compound, thionite compound, or thionate compound.

The present applicants found in a preliminary evaluative clinical trial with far-advanced human cancer patients having histologically verified malignancies representing a wide range of cancer types (leukemia, breast, colon, lung, prostate, larynx, testis, uterus, pancreas, muscle lymphoma, including lymphoma in the leg or gluteal muscles, carcinoma, sarcoma) that a significant rate and extent of reduction in tumor size occurred, often followed by complete remission. The therapy system of the present invention substantially avoids several of the well-known problems and limitations of conventional cancer chemotherapy such as development of resistant malignant-cell variations, excessive concomitant toxicity, dependence on phase of cell cycle and mutagenic side effects.

In other preliminary clinical trials, the present applicants surprisingly found clear evidence of the effectiveness of essentially the same therapy system when applied to patients afflicted with Down syndrome, hypercholesterolemia and cardiovascular disease.

### Conditions and disorders

The present invention relates to a novel method useful for preventing and treating cancer, AIDS, ARC (AIDS-related complex), cachexia secondary to AIDS, cachexia secondary to cancer, diabetes, Down syndrome, cardiovascular diseases, hypercholesterolemia, inflammatory gastrointestinal disorders, hyperproliferative diseases other than cancer, arthritis, metabolic syndrome, and neurodegenerative diseases. This novel method, useful for treating said disease conditions, comprises the delivery of therapeutically-effective amounts of the following compounds, either individually or intermixed: sulfide compounds, thiosulfate compounds, thionite compounds, thionate compounds, and any chemical species capable of providing to a subject in need thereof a sulfide compound, thionite compound, or thionate compound to the gut of a mammal in need thereof.

This invention further comprises treatment by "parenteral administration" of thiosulfate compounds and/or thionate compounds and/or thionite compounds and/or sulfide compounds and/or any chemical species capable of providing to a subject in need thereof a sulfide compound, thiosulfate compound, thionite compound, or thionate compound to said patient (or non-human mammal) afflicted with cancer, AIDS, ARC, cachexia secondary to cancer, cachexia secondary to AIDS, diabetes, Down syndrome, cardiovascular disease, inflammatory gastrointestinal diseases, hyperproliferative diseases other than cancer, arthritis, metabolic syndrome, or a neurodegenerative disease.

This invention further comprises treatment of a patient (or non-human mammal) afflicted with cancer, AIDS, ARC, cachexia secondary to cancer, cachexia secondary to AIDS, diabetes, Down syndrome, cardiovascular disease, inflammatory gastrointestinal diseases, hyperproliferative diseases other than cancer, arthritis, metabolic syndrome, or a neurodegenerative disease by administration of therapeutically effective amounts of at least one of the substances herein disclosed by any means that produces contact of the active agent or agents with their site of action in the mammal's body.

### Cancer

Conditions that may be treated using compositions of the invention include cancers (both solid tumors and hematological malignancies). Cancers are generally characterized by unregulated cell growth, formation of malignant tumors, and invasion to nearby parts of the body. Cancers may also spread to more distant parts of the body through the lymphatic system or bloodstream. Cancers may be a result of gene damage due to tobacco use, certain infections, radiation, lack of physical activity, obesity, and/or environmental pollutants. Cancers may also be a result of existing genetic faults within cells to cause diseases due to genetic heredity. Screenings may be used to detect cancers before any noticeable symptoms appear and treatment may be given to those who are at higher risks of developing cancers (e.g., people with a family history of cancers). Examples of screening techniques for cancer include but are not limited to physical examination, blood or urine tests, medical imaging, and/or genetic testing. Non-limiting examples of cancers include: bladder cancer, breast cancer, colon and rectal cancer, endometrial cancer, kidney or renal cell cancer, leukemia, lung cancer, melanoma, Non-Hodgkin lymphoma, pancreatic cancer, prostate cancer, ovarian cancer, stomach cancer, wasting disease (i.e., cachexia secondary to cancer, e.g., loss of weight, muscle atrophy, fatigue, weakness, and significant loss of appetite), and thyroid cancer.

### AIDS and ARC

The compositions of the invention are also useful for treating acquired immunodeficiency syndrome (AIDS)/human immunodeficiency virus infection (HIV) and AIDS-related complex (ARC). ARC is characterized by elevated or hyperactive B-cell humoral immune response compared to depressed or normal antibody reactivity in AIDS, follicular or mixed hyperplasia in ARC lymph nodes, leading to lymphocyte degeneration and depletion more typical of AIDS, and evolving succession of histopathological lesions such as localization of Kaposi's sarcoma. These symptoms signal the transition of subjects suffering from ARCS to AIDS.

The compositions of the invention can include preventative as well as active treatment of opportunistic infections resulting from HIV infection and can also be used to treat cachexia secondary to AIDS (e.g., loss of weight, muscle atrophy, fatigue, weakness, and significant loss of appetite).

### Diabetes

Compositions of the invention may also be useful for treating and preventing diabetes and its complications. Diabetes can be any metabolic disease in which a person has high blood sugar, either because the body does not produce enough insulin, or because cells do not respond to the insulin that is produced. Non-limiting examples of diabetes includes, type 1 diabetes mellitus, type 2 diabetes mellitus, gestational diabetes, congenital diabetes, cystic fibrosis-related diabetes, steroid diabetes, latent autoimmune diabetes of adults, and monogenic diabetes. Complications associated with diabetes include but are not limited to hypoglycemia, diabetic ketoacidosis, nonketotic hyperosmolar coma, cardiovascular disease, chronic renal failure, diabetic nephropathy, diabetic neuropathy, diabetes-related foot problems (e.g., diabetic foot ulcers), and diabetic retinopathy.

### Cardiovascular diseases

The compositions of the invention are also useful in treating and preventing cardiovascular diseases. As used herein cardiovascular diseases include, but are not limited to, arteriosclerosis, arrhythmias, coronary heart disease, ischemia, ischemia-reperfusion injury, endothelium dysfunction, in particular those dysfunctions affecting blood vessel elasticity, restenosis, thrombosis, angina, high blood pressure, cardiomyopathy, hypertensive heart disease, heart failure, cor pulmonale, cardiac dysrhythmias, endocarditis, inflammatory cardiomegaly, myocarditis, myocardial infarction, valvular heart disease, stroke and cerebrovascular disease, aortic valve stenosis, congestive heart failure, and peripheral arterial disease. In one aspect, the invention includes methods of administering the compositions for chronic treatment. In another aspect, the invention also includes methods of administering the compositions for acute treatment.

### Neurodegenerative diseases

Compositions of the invention may also be used to treat and prevent neurodegenerative diseases. Neurodegenerative diseases are any diseases that are characterized by the progressive loss of structure or function of neurons, including death of neurons. Neurodegenerative diseases may be caused by genetic mutations (e.g., CAG nucleotide triplet mutation), protein misfolding (e.g., aggregation of alpha-synuclein, hyperphosphorylated tau protein, and aggregation of beta amyloid), misregulation in protein degradation pathways (e.g., ubiquitin-proteasome pathway and autophagy-lysosome pathways), membrane damage, mitochondrial dysfunction, defects in axonal transport, and misregulation of programmed cell death pathways (e.g., apoptosis, autophagic, and cytoplasmic). Examples of neurodegenerative diseases include, but are not limited to Alzheimer's disease, Parkinson's disease, Huntington's disease, amyotrophic lateral sclerosis (ALS), Creutzfeldt-Jakob disease, primary progressive aphasia, progressive supranuclear palsy, spinocerebellar ataxia type 3, frontotemporal dementia, dementia with Lewy bodies, corticobasal degeneration, prion disorders, multiple system atrophy, hereditary spastic paraparesis, Friedreich's ataxia, childhood neurodevelopmental disorders, and amyloidoses.

### Other diseases and conditions

The therapeutic substances of the invention are also useful in treating other conditions such as: inflammatory gastrointestinal diseases (e.g., irritable bowel disease), hyperproliferative diseases other than cancer (e.g., liver disease, lung diseases, in particular, pulmonary edema and fibrosis, psoriasis), metabolic syndrome, anxiety, Benign Prostatic Hyperplasia (BPH), Carpal Tunnel Syndrome, bipolar disorder, cataracts, Celiacs Disease, Chronic Fatigue Syndrome, COPD, depression, fibromyalgia, GERD, glaucoma, hypertension, hyperthyroidism, influenza, kidney stones, lyme disease, sleep apnea, systemic lupus erythematosus (SLE), tinnitus, cirrhosis.

The therapeutic substances of the invention are also useful in preventing hair loss, in promoting hair growth, in increasing hair width, in reducing wrinkles, as a general anti aging drug, as a general well being drug, in wound healing, in neuropathy, in stopping fibrosis and callous formation, in nail growth, as a topical anti-inflammatory, protecting inner ear cilia, in muscle and cartilage growth, in amelioration, inhibition, and reversal of autoimmune diseases, in increasing nitric oxide production due to its antioxidant action, in increasing collagen production, in neuroprotection, in kidney protection, in decreasing antibody production in autoimmune diseases.

In general, the therapeutic substances can be used to prevent and treat diseases related to inhibition of inflammation and upregulation of prosurvival pathways. The therapeutic substances are also useful in preventing or minimizing damage (i.e., side effects) from medications that cause free radical production. The powerful anti-inflammatory, cytoprotective, immunomodulating, and trophic effects of the therapeutic substances on the vast majority of normal cells seem to be mediated mainly by its actions as an extremely versatile direct and indirect antioxidant and free radical scavenger.

### Medical Food

The present invention also relates to the therapeutic substances described herein as medical food for daily intake and for maintaining and promoting general health. In one aspect, the therapeutic substances of the invention are used as a paravitamin to provide a supplemental source of cysteine and its derivatives. Cysteine and its derivatives (e.g., glutathione, taurine, taurine conjugates with bile acids, hydrogen sulfide, and sulfate ions) play a role similar to that of vitamins. Like antioxidative vitamins, cysteine and its derivatives play a role in the oxidant/antioxidant balance and indirectly in the regulation of metabolic processes. Cysteine supplementation on top of the normal diet can have various beneficial effects, for example, cysteine supplementation can lead to an increase in muscle function, immune function, plasma albumin concentration and a decrease in TNF-α concentration. Supplementation can also restore the body's reservoirs of cysteine and glutathione levels which are driving forces behind multiple ageing-related processes.

In another aspect, the paravitamins are medical foods providing a minimum amount of calories and maximum amount of a bioavailable form of sulfur intended for humans not receiving enough sulfur in their diets.

### Pharmaceutical compositions and treatment methods

This invention also relates to pharmaceutical compositions comprising one or more of the above-mentioned substances and-optionally- and one or more pharmaceutically acceptable excipients, carriers, diluents or adjuvants.

A composition of the present invention can be administered by a variety of methods known in the art. As will be appreciated by the skilled artisan, the route and/or mode of administration will vary depending upon the desired results. The pharmaceutical compositions can be formulated for parenteral, intranasal, topical, oral, or local administration, such as by a transdermal means, for prophylactic and/or therapeutic treatment. The pharmaceutical compositions can be administered parenterally (e.g., by intravenous, intramuscular, or subcutaneous injection), or by oral ingestion, or by topical application or intraarticular injection at areas affected by the vascular or cancer condition. Additional routes of administration include intravascular, intra-arterial, intratumor, intraperitoneal, intraventricular, intraepidural, as well as nasal, ophthalmic, intrascleral, intraorbital, rectal, topical, or aerosol inhalation administration. Sustained release administration is also specifically included in the invention, by such means as depot injections or erodible implants or components. Thus, the invention provides compositions for "parenteral administration" that comprise the above mentioned agents dissolved, colloidally dispersed, or suspended in an acceptable carrier, which may be non aqueous or aqueous, e.g., water, buffered water, saline, PBS, and the like. The compositions may contain pharmaceutically acceptable auxiliary substances as required to approximate physiological conditions, such as pH adjusting and buffering agents, tonicity adjusting agents, wetting agents, detergents and the like.

The therapeutic and/or prophylactic composition may be in the form of a solution, colloidal dispersion, a suspension, an emulsion, an infusion device, or a delivery device for implantation or it may be presented as a dry powder to be used as such or to be reconstituted with water or another suitable vehicle before use. The composition can be in the form of a sachet, tablet, capsule (e.g., hard gelatin capsule and soft gelatin capsule), liquid, or sustained release tablet for oral administration; or a liquid for intravenous, intrathecal, subcutaneous or "parenteral administration"; or a cream or ointment for topical administration, or comprise a polymer or other sustained release vehicle for local administration.

Methods well known in the art for making formulations are found, for example, in "Remington: The Science and Practice of Pharmacy" (20th ed., ed. A.R. Gennaro AR., 2000, Lippincott Williams & Wilkins, Philadelphia, PA). Formulations for "parenteral administration" may, for example, contain excipients, sterile water, saline, polyalkylene glycols such as polyethylene glycol, oils of vegetable origin, or hydrogenated naphthalenes. Biocompatible, biodegradable lactide polymer, lactide/glycolide copolymer, or polyoxyethylene-polyoxypropylene copolymers may be used to control the release of the substances. Nanoparticulate formulations (e.g., biodegradable nanoparticles, solid lipid nanoparticles, liposomes) may be used to control the biodistribution of the substances. Other potentially useful delivery systems include ethylene-vinyl acetate copolymer particles, osmotic pumps, intrathecal pumps, implantable infusion systems, and liposomes. The concentration of the substance in the formulation varies depending upon a number of factors, including the dosage of the drug to be administered, and the route of administration.

Therapeutic and/or prophylactic compositions typically must be sterile and stable under the conditions of manufacture and storage. The composition can be formulated as a suspension, microemulsion, liposome, or other ordered structure suitable to high drug concentration. The carrier can be a solvent or dispersion medium containing, for example, water, ethanol, petroleum jelly (e.g., Vaseline®), polyol (e.g., glycerol, propylene glycol, and liquid polyethylene glycols, and the like), and suitable mixtures thereof, formulated at different percentages (e.g., 5, 10, 15, 20, 25, 30, 35, 40, 45, or 50% by weight in a dispersion medium described herein). The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, stearic acid salts and gelatin. Colloidal dispersions may be stabilized through addition of agents well known in the art.

The compositions of the invention may be sterilized by conventional sterilization techniques, or may be sterile filtered. The resulting aqueous dispersion may be packaged for use as is, or lyophilized, the lyophilized preparation being combined with a sterile aqueous carrier prior to administration. The pH of the preparations typically will be between 3 and 11, more preferably between 5 and 9 or between 6 and 8, and most preferably between 7 and 8, such as 7 to 7.5. The resulting compositions in solid or semisolid form may be packaged in multiple single dose units, each containing a fixed amount of the composition, such as in a sealed package of tablets or capsules. The composition in solid form can also be packaged in a sachet or a container for a flexible quantity, such as in a squeezable tube designed for a topically applicable cream or ointment.

Sterile injectable colloidal suspensions can be prepared by incorporating the active compound in the required amount in an appropriate solvent with one or a combination of ingredients enumerated above, as required, optionally followed by sterilization microfiltration. Generally, dispersions are prepared by incorporating the active compound into a sterile vehicle that contains a basic dispersion medium and the required other ingredients from those enumerated above. Dosage regimens are adjusted to provide the optimum desired response (e.g., a therapeutic and/or prophylactic response). For example, a single bolus may be administered, several divided doses may be administered over time or the dose may be proportionally reduced or increased as indicated by the exigencies of the therapeutic or prophylactic situation. For example, the compositions of the invention may be administered once or twice weekly by subcutaneous injection or once or twice monthly by subcutaneous injection.

It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic or prophylactic effect, optionally in association with the required pharmaceutical carrier. The specifications for the dosage unit forms of the invention are dictated by and directly dependent on (a) the unique characteristics of the active substance and the particular therapeutic or prophylactic effect to be achieved, and (b) the limitations inherent in the art of compounding such an active substance for the treatment of sensitivity in individuals.

When the substances of the present invention are administered as pharmaceuticals, to humans and animals, they can be given alone or as a pharmaceutical composition containing, for example, 1 to 100% (more preferably, 10 to 100%, such as 90 to 100%) of active ingredient, optionally in combination with one or more pharmaceutically acceptable carriers or excipients. To administer a composition of the invention by certain routes of administration, it may be necessary to coat the composition with, or co-administer the composition with a material to prevent its inactivation. For example, the composition may be administered to a subject in an appropriate carrier, for example, liposomes, an enteric carrier, or a diluent. Pharmaceutically acceptable diluents include saline and aqueous buffer solutions. Liposomes include water-in-oil-in-water CGF emulsions as well as conventional liposomes (Strejan et al., J. Neuroimmunol. 7:27-41, 1984). Pharmaceutically acceptable carriers include sterile aqueous solutions or dispersions and sterile powders for the extemporaneous preparation of sterile injectable colloidal solutions or dispersion. The use of such media and agents for pharmaceutically active substances is known in the art and is included in the invention except where any conventional media or agent is incompatible with the active substance. Examples of enteric carriers can include but are not limited to wet microcrystalline cellulose, wet powdered cellulose, methyl acrylate-methacrylic acid copolymers, cellulose acetate succinate, hydroxyl propyl methyl cellulose phthalate, hydroxyl propyl methyl cellulose acetate succinate, polyvinyl acetate phthalate, methyl methacrylate-methacrylic acid copolymers, sodium alginate, and stearic acid. Supplementary active substances can also be incorporated into the compositions.

Accordingly, this invention relates to a method for preparing pharmaceutically acceptable dosage forms containing the aforementioned ingredients and capable of releasing the pharmacologically active ingredient or ingredients in the gut of a mammal in need thereof. This invention further comprises the novel formulation of active sulfur substances for oral use, by combining the active ingredients with enteric carriers (e.g., those described herein). In the absence of an enteric carrier, some of the disclosed active compounds produce almost unbearable gastric distress upon reacting with acid in the stomach and concomitant release of large volumes of gaseous (and toxic) hydrogen sulfide. One novel enteric carrier is wet microcrystalline or powdered cellulose, which previously had been known as a disintegrant. Without wishing to be bound by theory, we believe that mixing highly reactive sulfides with wet cellulose (or other materials with the same characteristics) prevents the rapid release of the sulfides in the areas where they can produce damage as well as their almost instantaneous reaction with acid in the stomach with concomitant release of large volumes of gaseous hydrogen sulfide. Otherwise (as is usually the case) when an ordinary gelatin capsule containing drug and MCC reaches the stomach and gastric juice, which contains mainly water, contacts the dry MCC, this highly porous material (by "wicking action") rapidly absorbs gastric juice, disintegrates (as intended) and the capsule's contents are quickly released in the stomach. The wet cellulose, in contrast, being already wet absorbs significantly less water, thus remaining intact until it reaches the gut.

The present invention further comprises administering one of the formulations herein described to non-human mammals, i.e. as veterinary medication for treatment of said non-human mammals in need thereof, as well as for prophylactic purposes.

The applicant has demonstrated that delivery to a patient (or non-human mammal) afflicted with cancer, hypercholesterolemia/cardiovascular disease, or Down syndrome of safe and effective amounts of the compositions herein disclosed constitutes an effective treatment method.

In cancer patients, treatment in accordance with this invention will usually bring about a rapid and marked reduction of tumor size: such size reduction is characteristic clinical evidence for malignant cell death and degeneration (oncolysis); a similar reduction in malignant cell content of tissues containing disperse (nonaggregated) malignant cells will also usually result from treatment carried out as prescribed in this invention. In fact, dosage should be closely monitored to avoid any side effects due to either medication toxicity or massive release of toxins by malignant cell's lysis; it may be preferable to treat in short courses of several days, leaving a few days in between.

An effective amount of the herein described compositions can be administered for prophylactic or therapeutic treatments. In prophylactic applications, compositions can be administered to a subject with a clinically determined predisposition or increased susceptibility to development of cardiovascular diseases, cancer, diabetes, neurodegenerative diseases, AIDS, inflammatory gastrointestinal diseases, hyperproliferative diseases other than cancer, metabolic syndrome, and other pathological conditions associated with oxidative stress, an imbalance in redox homeostasis, chronic inflammation, and/or immune dysfunction. Compositions of the invention can be administered to the patient (e.g., a mammal, human, or non-human) in an amount sufficient to delay, reduce, or preferably prevent the onset of the clinical disease. In therapeutic applications, compositions are administered to a patient (e.g., a human) already suffering from a cardiovascular disease, cancer, diabetes, a neurodegenerative disease, AIDS, inflammatory gastrointestinal diseases, hyperproliferative diseases other than cancer, metabolic syndrome, and other pathological conditions associated with oxidative stress, an imbalance in redox homeostasis, and/or chronic inflammation, and/or immune dysfunction, in an amount sufficient to cure or at least partially arrest the symptoms of the condition and its complications. An amount adequate to accomplish this purpose is defined as a "therapeutically effective dose," an amount of a compound sufficient to substantially improve some symptom associated with a disease or a medical condition. For example, in the treatment of a cardiovascular disease, cancer, diabetes, a neurodegenerative disease, AIDS, inflammatory gastrointestinal diseases, hyperproliferative diseases other than cancer, metabolic syndrome, and other pathological conditions associated with oxidative stress, an imbalance in redox homeostasis, and/or chronic inflammation, and/or immune dysfunction, an agent or substance which decreases, prevents, delays, suppresses, or arrests any symptom of the disease or condition would be therapeutically effective. A therapeutically effective amount of an agent or substance is not required to cure a disease or condition but will provide a treatment for a disease or condition such that the onset of the disease or condition is delayed, hindered, or prevented, or the disease or condition symptoms are ameliorated, or the term of the disease or condition is changed or, for example, is less severe or recovery is accelerated in an individual.

In acute situations the patient or non-human mammal can be given a high initial "loading dose", followed by a 50% lower "maintenance dose."

In every instance close monitoring of the patient or non-human mammal is necessary, especially upon initial administration of any of the formulations herein disclosed, since a mild or severe allergic reaction (including anaphylactic shock) might ensue in susceptible individuals. Although such allergic reaction was not observed in any of the numerous patients treated thus far, it is a well-known fact that oral administration of sulfites and metabisulfites has provoked this kind of reaction (asthmatic episodes, for instance) in susceptible individuals.

Fortunately, sulfite susceptibility among the general population is probably very low, since "sulfite compounds" are widely used as "pharmaceutical aids" (antioxidants) in many types of dosage forms for oral administration ("The Merck Index" 12.sup.th Edition, monograph #8784, Merck and Co., 1996). However, it is also known that the prevalence of sulfite susceptibility among asthmatic patients is higher than among the general population; therefore it is advisable to ensure that patients in this "high risk" group be screened and declared not susceptible to orally-administered sulfites before treatment is instituted.

The compositions and formulations of the present invention may be used in combination with either conventional methods of treatment or therapy or may be used separately from conventional methods of treatment or therapy. When the substances and formulations of this invention are administered in combination therapies with other agents, they may be administered sequentially or concurrently to an individual. Alternatively, pharmaceutical compositions according to the present invention include a combination of a substance or formulation of the present invention optionally in association with a pharmaceutically acceptable excipient, as described herein, and another therapeutic or prophylactic agent known in the art.

The formulated agents can be packaged together as a kit. Non-limiting examples include kits that contain, e.g., two pills, a pill and a powder, a suppository and a liquid in a vial, two topical creams, etc. The kit can include optional components that aid in the administration of the unit dose to patients, such as vials for reconstituting powder forms, syringes for injection, customized IV delivery systems, inhalers, etc. Additionally, the unit dose kit can contain instructions for preparation and administration of the compositions. The kit may be manufactured as a single use unit dose for one patient, multiple uses for a particular patient (at a constant dose or in which the individual compounds may vary in potency as therapy progresses); or the kit may contain multiple doses suitable for administration to multiple patients ("bulk packaging"). The kit components may be assembled in cartons, blister packs, bottles, tubes, and the like.

### Dosage

It will be envisaged by those skilled in the art that the actual daily dosages of the foregoing compositions to be administered to a patient or to a non-human mammal will lie entirely within the discretion of the physician or veterinarian, as the case might be. Thus, the daily dosage for an adult human male of average weight (i.e. about 70 kg) should be greater than that for a child (or for a non-human mammal of lesser weight than the average human male) if other factors are equal, but the converse would be expected when dealing with e.g. either humans or non-human mammals heavier than the average human male.

Additionally, of course, the appropriate dosage administered in any given case will vary with the age, general health condition, nature and extent of symptoms and nature of concurrent treatment (if any). Further, the selected dosage level will depend upon a variety of pharmacokinetic factors including the activity of the particular compositions of the present invention employed, the route of administration, the time of administration, the rate of absorption of the particular agent being employed, the duration of the treatment, other drugs, substances, and/or materials used in combination with the particular compositions employed.

For example, the physician or veterinarian can start doses of the substances of the invention employed in the pharmaceutical composition at levels lower than that required in order to achieve the desired therapeutic effect and gradually increase the dosage until the desired effect is achieved. In general, a suitable daily dose of a composition of the invention will be that amount of the substance which is the lowest dose effective to produce a therapeutic effect. Such an effective dose will generally depend upon the factors described above. Preferably, the effective daily dose of a therapeutic and/or prophylactic composition may be administered as two, three, four, five, six or more sub-doses administered separately at appropriate intervals throughout the day, optionally, in unit dosage forms. In preferred embodiments, the unit dosage form is an acid-resistant capsule such as acid-armor capsule™, sold by Arthur Andrew Medical, Inc. or DRcaps acid-resistant capsule™, available from Capsugel Inc.

In all cases, treatment must be adjusted as required on the basis of frequent individual clinical evaluations, with due consideration of appropriate test results.

Preferred therapeutic dosage levels are between about 75 mg to about 2500 mg (e.g., 80, 90, 100, 125, 150, 175, 200, 225, 250, , 275, 300, 325, 350, 375, 400, 425, 450, 475, 500, 550, 575, 600, 625, 650, 675, 700, 725, 750, 775, 800, 825, 850, 875, 900, 925, 950, 975, 1000, 1025, 1050, 1075, 1100, 1150, 1200, 1250, 1300, 1350, 1400, 1450, 1500, 1550, 1600, 1650, 1700, or 1750, 1800, 1900, 2000, 2100, 2200, 2300, 2400, or 2500 mg) of active ingredient(s) (e.g., any of the active sulfur substances described herein) per day administered orally to adults of average weight afflicted with most of the symptoms, syndromes and pathological conditions described herein, Preferred prophylactic dosage levels are between about 75 mg to about 800 mg (e.g., 100, 150, 175, 200, 225, 250, 275, 300, 325, 350, 375, 400, 425, 450, 475, 500, 525, 550, 575, 600, 650, 700, or 750mg). In cancer, AIDS, and some chronic or refractory pathologies, the preferred oral dosage levels are 2000 mg per day or higher (e.g., 2450, 2500, 3000, 3500, 4000, 8000 mg, or 10 g) for an adult of average weight. For children afflicted with cancer, the dose may be titrated (e.g., the dose may be escalated gradually until signs of gastrointestinal toxicity appear, such as diarrhea or nausea). In preferred embodiments, the pharmaceutical compositions of the invention are extremely safe for oral administration and most patients can tolerate higher dosages as treatment progresses.

### Combination therapy

The present invention also comprises the use of combination therapies involving administration of the aforementioned active ingredients with the following active agents and/or compounds.

### Prevention drugs for cardiovascular diseases

Compositions of the invention can be administered in combination with one or more drugs that are used as secondary prevention drugs for cardiovascular diseases. Examples of preventative drugs include, but are not limited to, β blockers (e.g., nonselective agents, e.g., alprenolol, carteolol, oxprenolol, sotalol, timolol, e.g., β₁-selective agents, e.g., acebutolol, betaxolol, celiprolol, metoprolol, e.g., β₂-selective agents, e.g., butaxamine, e.g., β₃-selective agents, e.g., SR 59230A), statins (e.g., atorvastatin, cerivastatin, fluvastatin, lovastatin, mevastatin, pravastatin, simvastatin, and rosuvastatin), fibrates (e.g., bezafibrate, ciprofibrate, clofibrate, gemfibrozil, and fenofibrate), biguanides (e.g., metformin, phenformin, buformin, and proguanil), antihypertension agents, and/or ACE inhibitors (e.g., sulfhydryl-containing agents, e.g., captopril, zofenopril, e.g., dicarboxylate-containing agents, e.g., enalapril, ramipril, quinapril, perindopril, imidapril, e.g., phosphate-containing agents, e.g., fosinopril).

### Anti-neurodegenerative drugs

The composition of the invention can be administered in combination with one or more anti-neurodegenerative drugs. Examples of anti-neurodegenerative drugs include, but are not limited to, acetylcholinesterase inhibitors (e.g., donepezil, galantamine, and rivastigmine), anti-glutamate agent (e.g., amantadine, GABA-ergic, valproic acid), reserpine, tetrabenazine, typical/atypical neuroleptics, tricyclic antidepressants, SSRIs, carbamazepine, baclofen, tizanidine, hydergine, choline, piracetam, and lamotrigine.

### Anti-inflammatory drugs

The composition of the invention can be administered in combination with one or more anti-inflammatory drugs. Examples of anti-inflammatory drugs include, but are not limited to, steroids (e.g., glucocorticoids, e.g., corticosteroids), non-steroidal anti-inflammatory drugs (NSAIDs) (e.g., aspirin, diflunisal, salsalate, ibuprofen, naproxen, fenoprofen, ketoprofen, flurbiprofen, sulindac, etodolac, ketorolac, nabumetone, piroxicam, meloxicam, tenoxicam, mefenamic acid, flufenamic acid, tolfenamic acid, celecoxib, rofecoxib, parecoxib, etoricoxib, firocoxib, nimesulide, and licofelone), immune selective anti-inflammatory derivatives (ImSAIDs) (e.g., phenylalanine-glutamine-glycine (FEG) and its D-isomeric form (feG)), and/or herbs (e.g., Harpagophytum, hyssop, ginger, turmeric, Arnica montana, and willow bark)

### Dietary Supplements

The composition of the invention can be administered in combination with one or more dietary supplements to promote and/or maintain general health. Examples of dietary supplements include, but are not limited to, a vitamin (e.g., Vitamin A, Vitamin B₁, B₂, B₃, B₅, B₆, B₇, B₉, B₁₂, Vitamin C, Vitamin D, Coenzyme Q, Vitamin E, and Vitamin K), a mineral (e.g., potassium, chlorine, sodium, calcium, magnesium, phosphorus, zinc, iron, manganese, copper, iodine, selenium, and molybdenum), an herb or botanical (e.g., St. John's-wort, kava, Shilajit, and Chinese herbal medicines), an amino acid (e.g., glycine, serine, methionine, cysteine, aspartic acid, glutamic acid, glutamine, tryptophan, and phenylalanine), glucosamine, chondroitin sulfate, vinpocetine, pramiracetam, diallyl sulfide, a concentrate, constituent, extract, and/or a combination of any of the above, and/or micronutrients.

### Anticancer/anti-proliferative drugs

The composition of the invention can be formulated or administered in combination with one or more anticancer drugs. Examples of anticancer agents include, but are not limited to: chemotherapeutic agents (e.g., arsenic trioxide, cisplatin, carboplatin, chlorambucil, melphalan, nedaplatin, oxaliplatin, triplatin tetranitrate, satraplatin, imatinib, nilotinib, dasatinib, and radicicol), immunomodulatory agents (e.g., methotrexate, leflunomide, cyclophosphamide, cyclosporine A, minocycline, azathioprine, antibiotics (e.g., tacrolimus), methylprednisolone, corticosteroids, steroids, mycophenolate mofetil, rapamycin, mizoribine, deoxyspergualin, brequinar, T cell receptor modulators, and cytokine receptor modulators), antiangiogenic agents (e.g., bevacizumab, suramin, and etrathiomolybdate), mitotic inhibitors (e.g., paclitaxel, vinorelbine, docetaxel, abazitaxel, ixabepilone, larotaxel, ortataxel, tesetaxel, vinblastine, vincristine, vinflunine, and vindesine), nucleoside analogs (e.g., gemcitabine, azacitidine, capecitabine, carmofur, cladribine, clofarabine, cytarabine, decitabine, floxuridine, fludarabine, fluorouracil, mercaptopurine, pentostatin, tegafur, and thioguanine), DNA intercalating agents (e.g., doxorubicin, actinomycin, bleomycin, mitomycin, and plicamycin), topoisomerase inhibitors (e.g., irinotecan, aclarubicin, amrubicin, belotecan, camptothecin, daunorubicin, epirubicin, etoposide, idarubicin, mitoxantrone, pirarubicin, pixantrone, rubitecan, teniposide, topotecan, valrubicin, and zorubicin), folate antimetabolites (e.g., pemetrexed, aminopterin, methotrexate, pralatrexate, and raltitrexed), mitocans (e.g., sodium dichloroacetate and 3-bromopyruvic acid), and other targeting agents (e.g., agents that target particular enzymes or proteins involved in cancer or agents that target particular organs or types of cancers), and combinations thereof.

### Drugs for Metabolic Syndrome

The present invention also comprises the use of combination therapies with drugs used to treat or prevent metabolic syndrome. Drugs in this category should be tailored to target the specific components of the metabolic syndrome that are present in the patient. For example, cholesterol lowering drug classes include statins and fibrates. Blood pressure medications of various classes can also be used (e.g., aspirin to reduce cardiac risk and supplements such as omega 3-fatty acids and fish oils). Drugs used to treat type 2 diabetes such as Metformin has also been found to prevent onset of diabetes in people with metabolic syndrome.

### Drugs for treating of AIDS and other combination therapies

The present invention also comprises the use of combination therapies with immunostimulant(s) (e.g., prolactin, growth hormone, vitamin D, deoxycholic acid), hormone antagonist (s) and agonist(s) (e.g., triiodothyronine, insulin), antiviral agent(s) (e.g., gallic acid), drugs used in the treatment of AIDS (e.g., combination products, e.g., efavirenze, tenofovir, emtricitabine, rilpivirine, cobicistat; nucleoside reverse transcriptase inhibitors (NRTIs), e.g., lamivudine, abacavir, zalcitabine, disoproxil, fumarate, didanosine, ddC, AZT, ZDV; nonnucleoside reverse transcriptase inhibitors (NNRTIs), e.g., rilpivirine, etravirine, delavirdine, nevirapine; protease inhibitors, e.g., amprenavir, tipranavir, saquinavir; fusion inhibitors; CCR5 co-receptor antagonists; and HIV integrase strand transfer inhibitors), and antibiotics.

### EXAMPLES

The invention can be illustrated by the following non-limiting examples. These examples are set forth merely for illustrative purposes and many other variations may be used.

### Formulation Example 1

Two hundred and fourteen (214) parts by weight sodium hydrogen sulfide (NaSH), six hundred and forty (640) parts by weight distilled water and two thousand (2000) parts by weight food grade microcrystalline cellulose were thoroughly blended at room temperature. The final powdery mixture was used for filling standard, two-piece hard gelatin capsules with 1,000 milligrams per capsule.

### Formulation Example 2

Three hundred and seventy two (372) parts by weight sodium thiosulfate (Na2S2O3), six hundred and forty (640) parts by weight distilled water and two thousand (2000) parts by weight food grade microcrystalline cellulose were thoroughly blended at room temperature. The resulting powdery mixture was used for filling standard two-piece hard gelatin capsules with 1,000 mg per capsule.

### Formulation Example 3

Four hundred and sixty four (464) parts by weight potassium metabisulfite (K2S2O5), six hundred and forty (640) parts by weight distilled water and 2,000 parts by weight food grade microcrystalline cellulose were thoroughly blended at room temperature. The resulting powdery mixture was used for filling standard, two-piece hard gelatin capsules with 1,000 mg per capsule.

### Formulation Example 4

One-thousand five-hundred and thirty-two (1532) parts by weight sodium thiosulfate (Na2S2O3), two-hundred and thirty-two (232) parts by weight potassium metabisulfite, two-hundred and twelve (212) parts by weight sodium metabisulfite, four hundred and eighteen (418) parts by weight sodium sulfide nonahydrate one thousand and ninety (1090) parts by weight distilled water and two thousand (2000) parts by weight food-grade microcrystalline cellulose were thoroughly blended at room temperature. The resulting powdery mixture was used for filling standard, two-piece hard gelatin capsules with 1,000 milligrams per capsule.

### Utility Example 1

Patient: Male, 74 year-old.

Baseline condition: Scarcely differentiated epidermoid larynx carcinoma associated with severe necrosis. The tumor is not operable on account of patient's marked cachexia and critical cardiovascular condition. Patient's status diagnosed as terminal.

Treatment regime: Administration of 24 capsules per day, each containing 1000 mg. of a formulation prepared as in "formulation example 1" (approximately 75 mg. of active ingredient and 925 mg of inert ingredients).

Treatment outcome: At the end of the first week, tumor size was halved; at the end of the third week, tumor size was one fourth of original size.

### Utility Example 2

Patient: Male, 15 year-old.

Baseline condition: Acute lymphocytic leukemia refractory to conventional chemotherapy. Patient's status diagnosed as terminal.

Treatment regime: Administration of 18 capsules per day, each containing 1000 mg of a mixture prepared as in "formulation example 2" (approximately 125 mg of active ingredient and 875 mg of inert ingredients).

Treatment outcome: At the end of the second week, patient's blood count (leucocytes, erythrocytes and platelets) presented an alarming reduction, associated with a critical condition that required several blood transfusions. At the end of the third week patient's condition was stable, presenting a normal blood count and a reduction in blast count from 90% to 38%. At the end of the fourth week, all blood parameters were normal (including a zero blast count). Patient's status diagnosed as normal with total remission.

### Utility Example 3

Patient: Male, 38 year-old.

Baseline condition: Seminoma refractory to radiotherapy and to conventional chemotherapy.

Treatment regime: Administration of 30 capsules per day, each containing 1000 mg of the mixture prepared as in "formulation example 3" (approximately 150 mg of active ingredient and 850 mg of inert ingredients).

Treatment outcome: At the end of the first week, the persistent pain in the remaining testicle disappeared and the consistency of the testicle was almost normal. At the end of the sixth month patient's status was diagnosed as normal with total remission.

### Utility Example 4

Patient: Male, 63 year-old.

Baseline condition: Colon carcinoma. The tumor's size precludes surgery. Patient's status diagnosed as terminal.

Treatment regime: Administration of 24 capsules per day, each containing 1000 mg of a formulation prepared as in "formulation example 4" (approximately 280 mg of sodium thiosulfate, 42 mg of potassium metabisulfite, 39 mg of sodium metabisulfite, 76 mg of sodium sulfide nonahydrate and 563 mg of inert ingredients).

Treatment outcome: At the end of the second month, the tumor had disappeared. Patient's status was diagnosed as normal with total remission. Patient is still in good health 6 months after remission.

### Utility Example 5

Patient: Male, 15 year-old

Baseline condition: Testis carcinoma with bone (spine) metastasis. Patient's status diagnosed as terminal.

Treatment regime: Administration of 18 capsules per day, each containing 1000 mg of a formulation prepared as in "formulation example 4" (approximately 280 mg of sodium thiosulfate, 42 mg of potassium metabisulfite, 39 mg of sodium metabisulfite, 76 mg of sodium sulfide nonahydrate and 563 mg of inert ingredients).

Treatment outcome: At the end of the second week all subjective symptoms (pain, chronic fatigue, etc.) had disappeared. At the end of the sixth week the spine tumor had disappeared. Patient decided (on his own) to discontinue treatment and did so during the seventh, eighth, and ninth weeks. At the end of the ninth week, alarming symptoms forced the patient to seek help again: a CAT-scan showed the presence of two new tumors (one on a different spine location and the other in the previously unaffected testis) and the treatment was reinstituted with marked abatement of subjective cancer symptoms. When patient was confronted with the need to surgically ablate the previously unaffected testis, he refused and again discontinued treatment. Patient died two weeks after discontinuing treatment.

### Utility Example 6

Patient: Male, 4 year-old.

Baseline condition: Rhabdomyosarcoma of the nasopharynx, phase 4, refractory to radiotherapy and conventional chemotherapy. Patient's status diagnosed as terminal.

Treatment regime: 10 capsules per day, each containing 1000 mg of a formulation prepared as in "formulation example 4."

Treatment outcome: At the end of the second week a CAT-scan showed a halving in tumor size. At the end of the first month, a CAT-scan showed a 75% decrease in tumor size. At the end of the second month, a CAT-scan showed an 85% decrease in tumor size. Patient is now asymptomatic.

### Utility Example 7

Patient: Male, 68 year-old.

Baseline condition: Lung carcinoma (phase 4) with bone (clavicle) metastasis, refractory to conventional chemotherapy. Patient's status diagnosed as terminal, with a life expectancy of at best two weeks.

Treatment regime: 16 capsules per day, each containing 1000 mg of a formulation prepared as in "formulation example 4."

Treatment outcome: At the end of the 12th week, a CAT-scan showed no tumor growth. Since then, the patient has been asymptomatic for 5 months.

### Utility Example 8

Patient: Female, 44 year-old.

Baseline condition: Breast adenocarcinoma, metastasized to bone and lymph nodes with the patient refusing to submit to either surgery or conventional chemotherapy.

Treatment regime: 20 capsules per day, each containing 1000 mg of a formulation prepared as in "formulation example 4."

Treatment outcome: At the end of the second week, a CAT-scan showed a 50% reduction in tumor size. At the end of the first month, a CAT-scan showed a 90% reduction in tumor size. At the end of the second month, patient status was diagnosed as normal with complete cancer remission.

### Utility Example 9

Patient: Female, 55 year-old

Baseline condition: Uterine corpus carcinoma, phase 4. Patient's status diagnosed as terminal.

Treatment regime: 40 capsules per day, each containing 1000 mg of a formulation prepared as in "formulation example 4."

Treatment outcome: At the end of the third month, patient status was diagnosed as normal with complete cancer remission.

### Utility Example 10

Patient: Male, 75 year-old

Baseline condition: Prostatic adenocarcinoma.

Treatment regime: 20 capsules per day, each containing 1000 mg of a formulation prepared as in "formulation example 4."

Treatment outcome: At the end of the sixth week, patient status was diagnosed as normal with complete cancer remission.

### Utility Example 11

Patient: Female, 26 year-old.

Baseline condition: Gluteal cancer, phase 4, refractory to radiotherapy and to conventional chemotherapy. Patient's status was diagnosed as terminal after five unsuccessful attempts at tumor removal by surgery. Patient complained of continuous excruciating pain, not amenable to treatment with analgesics.

Treatment regime: 18 capsules per day, each containing 1000 mg of a formulation prepared as in "formulation example 4."

Treatment outcome: At the end of the third day the excruciating pain subsided, leaving behind a sensation of discomfort. At the end of the first week, both the pain and the discomfort had disappeared completely. At the end of the 6th week there was evidence of massive tumor necrosis and concomitant reduction in tumor size.

### Utility Example 12

Patient: Female, 49 year-old.

Baseline condition: Leg liposarcoma, still present after several unsuccessful attempts at tumor removal by surgery.

Treatment regime: 20 capsules per day, each containing 1000 mg of a formulation prepared as in "formulation example 4."

Treatment outcome: At the end of the sixth month, patient status was diagnosed as normal with complete cancer remission.

### Utility Example 13

Patient: Male, 42 year-old

Baseline condition: Adenocarcinoma of the pancreas, phase 4. Patient status diagnosed as terminal.

Treatment regime: 25 capsules per day, each containing 1000 mg of a formulation prepared as in "formulation example 4."

Treatment outcome: At the end of the second month, patient status was diagnosed as normal with complete cancer remission.

### Utility Example 14

Patient: Female, 34 year-old

Baseline condition: Multiple myeloma.

Treatment regime: 18 capsules per day, each containing 1000 mg of a formulation prepared as in "formulation example 4."

Treatment outcome: At the end of the second month, complete remission was observed.

### Utility Example 15

Patient: Male, 58 year-old

Baseline condition: Hypercholesterolemia (total serum cholesterol 900 mg/dL).

Treatment regime: Administration of 20 capsules per day, each containing 1000 mg of a mixture prepared as in "formulation example 4."

Treatment outcome: At the end of the sixth week total blood cholesterol level was 200 mg/dL

### Utility Example 16

Patient: Male, 63 year-old

Baseline condition: Hypercholesterolemia (total serum cholesterol 700 mg/dL).

Treatment regime: Administration of 20 capsules per day, each containing 1000 mg of a mixture prepared as in "formulation example 4."

Treatment outcome: At the end of the sixth week, total blood cholesterol level was 248 mg/dL.

### Utility Example 17

Patient: Male, 74 year-old

Baseline condition: Hypercholesterolemia (total serum cholesterol 490 mg/dL).

Treatment regime: Administration of 20 capsules per day, each containing 1000 mg of a mixture prepared as in "formulation example 4."

Treatment outcome: At the end of the sixth week, total blood cholesterol level was 215 mg/dL.

### Utility Example 18

Patient: Male, 76 year-old

Baseline condition: Hypercholesterolemia (total serum cholesterol 618 mg/dL).

Treatment regime: Administration of 20 capsules per day, each containing 1000 mg of a mixture prepared as in "formulation example 4."

Treatment outcome: At the end of the sixth week, total blood cholesterol level was 195 mg/dL.

### Utility Example 19

Patient: Female, 56 year-old

Baseline condition: Hypercholesterolemia (total serum cholesterol 514 mg/dL).

Treatment regime: Administration of 20 capsules per day, each containing 1000 mg of a mixture prepared as in "formulation example 4."

Treatment outcome: At the end of the sixth week, total blood cholesterol level was 202 mg/dL.

### Utility Example 20

Patient: Male, 50 year-old

Baseline condition: Hypercholesterolemia (total serum cholesterol 883 mg/dL).

Treatment regime: Administration of 20 capsules per day, each containing 1000 mg of a mixture prepared as in "formulation example 4."

Treatment outcome: At the end of the sixth week, total blood cholesterol level was 206 mg/dL.

### Utility Example 21

Patient: Female, 58 year-old

Baseline condition: Hypercholesterolemia (total serum cholesterol 300 mg/dL).

Treatment regime: Administration of 20 capsules per day, each containing 1000 mg of a mixture prepared as in "formulation example 4."

Treatment outcome: At the end of the sixth week, total blood cholesterol level was 200 mg/dL.

### Utility Example 22

Patient: Male, 63 year-old

Baseline condition: Hypercholesterolemia (total serum cholesterol 472 mg/dL).

Treatment regime: Administration of 20 capsules per day, each containing 1000 mg of a mixture prepared as in "formulation example 4."

Treatment outcome: At the end of the sixth week, total blood cholesterol level was 218 mg/dL.

### Utility Example 23

Patient: Female, 3 year-old

Baseline condition: Down syndrome with severe mental retardation Treatment regime: Administration of 10 capsules per day, each containing 1000 mg of a mixture prepared as in "formulation

### example 4."

Treatment outcome: At the end of the tenth day, cognitive ability had improved significantly as evidenced by an increased attention span, and development of verbal skills. Additionally there were improvements in sphincter control and muscle tone. At the end of the sixth month, cognitive development had reached a level similar to that of a normal 2 year old girl.

### Utility Example 24

Patient: Female, 3 year-old

Baseline condition: Down syndrome with severe mental retardation and joint hyperflexibility.

Treatment regime: Administration of 10 capsules per day, each containing 1000 mg of a mixture prepared as in "formulation example 4" plus glucosamine sulfate (oral, 1500 milligrams per day), plus chondroitin sulfate (oral, 1200 milligrams per day).

Treatment outcome: At the end of the 14th day, cognitive ability had improved significantly as evidenced by an increased attention span and development of verbal skills. Additionally there were improvements in sphincter control and muscle tone. At the end of the sixth month, cognitive development had reached a level similar to that of a normal 2 year old girl. Additionally, a reduction in joint hyperflexibility was observed.

### Utility Example 25

Patient: Female, 3 year-old

Baseline condition: Down syndrome with severe mental retardation

Treatment regime: Administration of 10 capsules per day, each containing 1000 mg of a mixture prepared as in "formulation example 4" plus coenzyme Q10 (oral, 300 milligrams per day).

Treatment outcome: At the end of the tenth day, cognitive ability had improved significantly as evidenced by an increased attention span and development of verbal skills. Additionally there were improvements in sphincter control and muscle tone. At the end of the fifth month, cognitive development had reached a level similar to that of a normal two-and-a-half year old girl.

### Utility Example 26

Patient: Female, 4 year-old

Baseline condition: Down syndrome with severe mental retardation.

Treatment regime: Administration of 10 capsules per day, each containing 1000 mg of a mixture prepared as in "formulation example 4."

Treatment outcome: At the end of the 10th day, cognitive ability had improved significantly as evidenced by an increased attention span and development of verbal skills. Additionally there were improvements in sphincter control and muscle tone. At the end of the twelfth month, cognitive development had reached a level similar to that of a normal 4 year old girl.

### Utility Example 27

Patient: Female, 4 year-old

Baseline condition: Down syndrome with severe mental retardation.

Treatment regime: Administration of 10 capsules per day, each containing 1000 mg of a mixture prepared as in "formulation example 4" plus multivitamins.

Treatment outcome: At the end of the 10th day, cognitive ability had improved significantly as evidenced by an increased attention span and development of verbal skills. Additionally there were improvements in sphincter control and muscle tone. At the end of the tenth month, cognitive development had reached a level similar to that of a normal 4 year old girl.

### Utility Example 28

Patient: Female, 4 year-old

Baseline condition: Down syndrome with severe mental retardation.

Treatment regime: Administration of 10 capsules per day, each containing 1000 mg of a mixture prepared as in "formulation example 4" plus choline (oral, 1000 milligrams per day) plus piracetam (oral, 1000 milligrams per day) plus pramiracetam (oral, 600 milligrams per day) plus selegiline (oral, 1 milligram per day), plus vinpocetine (oral, 5 milligrams per day), plus hydergine (oral, 5 milligrams per day).

Treatment outcome: At the end of the 10th day, cognitive ability had improved significantly as evidenced by an increased attention span and development of verbal skills. Additionally there were improvements in sphincter control and muscle tone. At the end of the seventh month, cognitive development had reached a level similar to that of a normal 4 year old girl.

### Utility Example 29

Patient: Female, 4 year-old

Baseline condition: Down syndrome with severe mental retardation and hypothyroidism.

Treatment regime: Administration of 10 capsules per day, each containing 1000 mg of a mixture prepared as in "formulation example 4" plus triiodothyronine (oral, 10 mcg per day).

Treatment outcome: At the end of the 10th day, cognitive ability had improved significantly as evidenced by an increased attention span and development of verbal skills. Additionally there were improvements in sphincter control and muscle tone. At the end of the eleventh month, cognitive development had reached a level similar to that of a normal 4 year old girl.

### Utility Example 30

Patient: Female, 4 year-old

Baseline condition: Down syndrome with severe mental retardation Treatment regime: Administration of 10 capsules per day, each containing 1000 mg of a mixture prepared as in "formulation example 4" plus triiodothyronine (oral, 10 mcg per day).

Treatment outcome: At the end of the 10th day, cognitive ability had improved significantly as evidenced by an increased attention span and development of verbal skills. Additionally there were improvements in sphincter control and muscle tone. At the end of the tenth month, cognitive development had reached a level similar to that of a normal 4 year old girl.

### Utility Example 31

Patient: Female, 4 year-old

Baseline condition: Down syndrome with severe mental retardation

Treatment regime: Administration of 10 capsules per day, each containing 1000 mg of a mixture prepared as in "formulation example 4" plus coenzyme Q10 (oral, 300 milligrams per day) plus triiodothyronine (oral, 10 mcg per day) plus choline (oral, 1000 milligrams per day) plus piracetam (oral, 1000 milligrams per day) plus pramiracetam (oral, 600 milligrams per day) plus niar (oral, 1 milligrams per day) plus hydergine (oral, 5 milligrams per day) plus vinpocetine (oral, 5 milligrams per day)

Treatment outcome: At the end of the 10th day, cognitive ability had improved significantly as evidenced by an increased attention span and development of verbal skills. Additionally there were improvements in sphincter control and muscle tone. At the end of the fifth month, cognitive development had reached a level similar to that of a normal 4 year old girl.

### Utility Example 32

Patient: Male, 68 year-old

Baseline condition: Patient confined to bed after several episodes of acute myocardial infarction. Patient status diagnosed as terminal.

Treatment regime: Administration of 20 capsules per day, each containing 1000 mg of a mixture prepared as in "formulation example 4" plus coenzyme Q10 (oral, 1000 milligrams per day).

Treatment outcome: At the end of the third month patient blood pressure was normal and he was able to walk and exercise moderately.

### Other Embodiments

While the invention has been described in connection with specific embodiments thereof, it will be understood that it is capable of further modification and that this patent application is intended to cover any variations, uses or adaptations following, in general, the principles of the invention and including such departures from the present disclosure as come within the ordinary skill of the art to which the invention pertains, and as may be applied to the essential features hereinbefore set forth, within the spirit of the invention.

## Claims

1. A pharmaceutical composition in unit dosage form for oral administration, said composition comprising an active sulfur substance, wherein said active sulfur substance is capable of providing to a subject in need thereof a therapeutically effective or prophylactically effective amount of a sulfide compound, thionite compound, thionate compound, thiosulfate compound, or a hydrate, salt, or mixture thereof; and optionally an enteric carrier.

2. The composition of claim 1, said composition comprising:
an active sulfur substance selected from the group consisting of a sulfide compound, thionite compound, thionate compound, thiosulfate compound, or a hydrate, salt, or mixture thereof; and
an enteric carrier in capsule form capable of delivering said active sulfur substance to the lower gastrointestinal tract of a subject upon ingestion without substantial release of said active sulfur substance in the stomach of said subject.

3. The composition of claim 1, wherein said active sulfur substances are present in an amount from about 50 mg to about 1500 mg.

4. The composition of claim 1, wherein said enteric carrier is selected from the group consisting of: wet microcrystalline cellulose, wet powdered cellulose, hypromellose, cellulose acetate-phthalate, gellan gum, methyl acrylate-methacrylic acid copolymers, cellulose acetate succinate, hydroxyl propyl methyl cellulose phthalate, hydroxyl propyl methyl cellulose acetate succinate, polyvinyl acetate phthalate, methyl methacrylate-methacrylic acid copolymers, sodium alginate, and stearic acid.

5. The composition of claim 1, wherein said composition comprises about 214 parts by weight sodium hydrogen sulfide (NaSH), about 640 parts by weight distilled water, and about 2000 parts by weight food grade microcrystalline cellulose.

6. The composition of claim 1, wherein said composition comprises about 372 parts by weight sodium thiosulfate (Na₂S₂O₃), about 640 parts by weight distilled water, and about 2000 parts by weight food grade microcrystalline cellulose.

7. The composition of claim 1, wherein said unit dosage form is a hard gelatin capsule.

8. The composition of claim 1, wherein said unit dosage form is an acid-resistant capsule selected from the group consisting of: acid-armor capsules™ and DRcaps acid-resistant capsules™.

9. The composition of claim 1, further comprising a third agent.

10. The composition of claim 9, wherein said third agent is selected from the group consisting of vitamins, micronutrients, coenzyme Q10, glucosamine, chondroitin sulfate, triiodothyronine, vinpocetine, pramiracetam, piracetam, hydergine, choline, niar, gallic acid, diallyl sulfide, anti-cancer agents, immunostimulants, antibiotics, hormone antagonists, antiviral agents, antihypertension agents, insulin, and anti-inflammatory agents.
